# EUROPEAN PATENT APPLICATION

(11) **EP 3 904 384 A1**
(43) Date of publication of application: **03.11.2021**
(21) Application number: 19889662.3
(22) Date of filing: 29.11.2019
(51) Int. Cl.: C07K 16/28, C07K 19/00, C12N 15/13, C12N 15/63, A61K 39/395

(54) **FULLY HUMANIZED ANTI-GITR ANTIBODY AND PREPARATION METHOD THEREFOR**

(30) Priority: 29.11.2018 CN 201811444696
(71) Applicant: Shanghai Pharmaexplorer Co., Ltd., Shanghai 201210 (CN); Pharmaexplorer Limited, Road Town, Tortola (VG)
(72) Inventor: QI, Qianqian, Shanghai 201210 (CN); DUAN, Qing, Shanghai 201210 (CN); LIU, Lile, Shanghai 201210 (CN); YANG, Tatchi Teddy, Shanghai 201210 (CN); LIU, Hu, Shanghai 201210 (CN); MA, Hui, Shanghai 201210 (CN); YANG, Xinxiu, Shanghai 201210 (CN); WANG, Dongxu, Shanghai 201210 (CN); DAI, Chaohui, Shanghai 201210 (CN); WANG, Mengying, Shanghai 201210 (CN)
(74) Representative: Rondano, Davide
(86) International application number: PCT/CN2019/122134
(87) International publication number: WO 2020/108636

(57) **Abstract**

Disclosed are a GITR-targeting antibody, a preparation method therefor and the use thereof. In particular, disclosed is a novel GITR-targeting chimeric antibody or a fully humanized monoclonal antibody. Also disclosed is a method for preparing the monoclonal antibody. The monoclonal antibody of the present invention can bind to a GITR antigen with a high specificity, and has a very high affinity and a significant anti-tumor activity or the like.

## Description

### Technical field

The invention relates to the field of biomedicine, in particular to a GITR antibody and a preparation method and application thereof.

### Background

Immune checkpoints refer to some inhibitory signal pathways in the immune system, which prevent tissue damage by regulating the persistence and intensity of immune responses in peripheral tissues, and participate in maintaining tolerance to self-antigens. Immune checkpoint therapy is a therapeutic method to improve tumor immune response by regulating T cell activity through a series of ways such as co-inhibition or co-stimulation signals. Targeted blocking immune checkpoint therapy is gradually becoming one of the effective strategies for anti-tumor immunity.

Inhibitors of immune checkpoint proteins/antibodies have the potential to treat various tumor types (such as metastatic melanoma, lung cancer, breast cancer, renal cell carcinoma, etc.). In the past few years, great breakthroughs have been made in molecular therapy of targeted immune checkpoints, showing great clinical application value. As a kind of immunosuppressive molecules, immune checkpoint molecules can avoid the damage and destruction of normal tissues by regulating the intensity and breadth of immune response, and play a key role in the occurrence and development of various diseases such as tumors. To block the action of immune checkpoint molecules to relieve the immune suppression in tumor patients is the basic idea of targeted immune checkpoint molecules in tumor therapy. At present, CTLA-4 and PD-1/PD-L1 are the most popular immune checkpoint molecules for research and application. However, there are few studies on GITR, a new target.

Tumor necrosis factor superfamily receptor glucocorticoid-induced TNFR-related protein (GITR) is a homodimer transmembrane glycoprotein with a molecular weight of 70KD. Its extracellular domain is rich in cysteine, and it is a stimulatory immunomodulatory receptor that can enhance cellular and humoral immunity. GITR is expressed in various immune cell subsets, including T cells, natural killing cells, B cells and regulatory T cells, with the highest expression on regulatory T cells (Tregs). There are many types of immunosuppressive cells in malignant tumor environment. At present, it is considered that Tregs play an extremely important role in the occurrence and development of tumors. Studies have shown that decreased Tregs function and/or transport can improve the survival rate of preclinical models of glioma. Therefore, taking Tregs and immunosuppressor molecules as targets, eliminating Tregs, controlling the number and function of Tregs, and enhancing the body's immune response to tumor provide a new idea for tumor immunotherapy. Other studies have shown that humanized anti-GITR antibodies have the function of improving the proliferation of naive and tumor infiltrating T cells, reducing the induction and inhibition of Tregs, and participating in triggering the phosphorylation of NF-κB in regulatory T cells and effector T cells.

The key role played by GITR in immune response has made it an increasingly attractive target for immunotherapy, including inducing or enhancing immune response against required tumor antigens or pathogenic antigens (such as viruses and other pathogenic organisms). The biological function of GITR makes it a potential target for the treatment of various cancers, so GITR has the function of activating the immune system. Therefore, anti-GITR antibodies have good effects in treating various cancers and infectious diseases.

Based on the current small quantity of GITR antibodies in research, there is an urgent need to develop GITR antibodies with better activity, wide indications and high yields to further improve the therapeutic and detection effects.

### Summary of the invention

In order to develop GITR antibodies with good activity, wide indications and high yields, the present invention provides a GITR antibody with high affinity and strong specificity and a preparation method thereof.

In a first aspect of the invention, it provides a heavy chain variable region of an antibody, wherein the heavy chain variable region comprises complementary determining regions or CDRs selected from the group consisting of:
VH-CDR1 shown in SEQ ID NO: 8n+2,
VH-CDR2 shown in SEQ ID NO: 8n+3, and
VH-CDR3 shown in SEQ ID NO: 8n+4;
wherein, each n is independently 0, 1, 2 or 3;
wherein, any one of the above amino acid sequences also includes a derivative sequence that is optionally with at least one amino acid added, deleted, modified, and/or substituted, and is capable of retaining the binding affinity to GITR.

In another preferred embodiment, the VH-CDR3 has the amino acid sequence shown in SEQ ID NO: 43.

In another preferred embodiment, the VH-CDR3 has the amino acid sequence shown in SEQ ID NO: 45.

In another preferred embodiment, the heavy chain variable region has the amino acid sequence shown in SEQ ID NO: 8n+1, wherein n is 0, 1, 2 or 3.

In another preferred embodiment, the substitution is the mutation of Serine S at position 84 to Asparagine N, the mutation of Valine V at position 86 to Leucine L and the mutation of Proline P at position 88 to Alanine A, wherein the positions are of SEQ ID NO: 1.

In another preferred embodiment, the substitution is the mutation of Serine S at position 84 to Asparagine N, the mutation of Valine V at position 86 to Leucine L, the mutation of Proline P at position 88 to Alanine A and the mutation of Aspartic D at position 103 to Glutamate E, wherein the positions are of SEQ ID NO: 1.

In another preferred embodiment, the substitution is the mutation of Serine S at position 84 to Asparagine N, the mutation of Valine V at position 86 to Leucine L, the mutation of Proline P at position 88 to Alanine A and the mutation of Glycine G at position 104 to Alanine A.

In another preferred embodiment, the substitution is the mutation of asparagine N at position 85 of SEQ ID NO: 25 to serine S.

In another preferred embodiment, the heavy chain variable region has the amino acid sequence shown in SEQ ID NO: 41.

In another preferred embodiment, the heavy chain variable region has the amino acid sequence shown in SEQ ID NO: 42.

In another preferred embodiment, the heavy chain variable region has the amino acid sequence shown in SEQ ID NO: 44.

In another preferred embodiment, the heavy chain variable region has the amino acid sequence shown in SEQ ID NO: 46.

In a second aspect of the present invention, it provides a heavy chain of an antibody, wherein the heavy chain comprises the heavy chain variable region according to the first aspect of the present invention.

In another preferred embodiment, the heavy chain further comprises a heavy chain constant region.

In another preferred embodiment, the heavy chain constant region is of human or murine origin.

In a third aspect of the present invention, it provides a light chain variable region of an antibody, wherein the light chain variable region comprises complementary determining regions or CDRs selected from the group consisting of:
VL-CDR1 shown in SEQ ID NO: 8n+6,
VL-CDR2 shown in SEQ ID NO: 8n+7, and
VL-CDR3 shown in SEQ ID NO: 8n+8;
wherein, each n is independently 0, 1, 2 or 3;
wherein, any one of the above amino acid sequences also includes a derivative sequence that is optionally with at least one amino acid added, deleted, modified, and/or substituted, and is capable of retaining the binding affinity to GITR.

In another preferred embodiment, the light chain variable region has the amino acid sequence shown in SEQ ID NO: 8n+5, wherein n is 0, 1, 2 or 3.

In another preferred embodiment, the substitution is the mutation of cysteine C at position 87 of SEQ ID NO: 13 to tyrosine Y.

In another preferred embodiment, the substitution is the mutation of cysteine C at position 87 of SEQ ID NO: 21 to tyrosine Y.

In another preferred embodiment, the substitution is the mutation of threonine T at position 42 to lysine K and the mutation of cysteine C at position 87 to tyrosine Y, wherein the positions are of SEQ ID NO: 29.

In another preferred embodiment, the light chain variable region has the amino acid sequence shown in SEQ ID NO: 47.

In another preferred embodiment, the light chain variable region has the amino acid sequence shown in SEQ ID NO: 48.

In another preferred embodiment, the light chain variable region has the amino acid sequence shown in SEQ ID NO: 49.

In a fourth aspect of the present invention, it provides a light chain of an antibody, wherein the light chain comprises the light chain variable region according to the third aspect of the present invention.

In another preferred embodiment, the light chain further comprises a light chain constant region.

In another preferred embodiment, the light chain constant region is of human or murine origin.

In a fifth aspect of the present invention, it provides an antibody having:
(1) the heavy chain variable region according to the first aspect of the present invention; and/or
(2) the light chain variable region according to the third aspect of the present invention;
or the antibody comprises: the heavy chain according to the second aspect of the present invention; and/or the light chain according to the fourth aspect of the present invention,
wherein, any one of the above amino acid sequences also includes a derivative sequence that is optionally with at least one amino acid added, deleted, modified, and/or substituted, and is capable of retaining the binding affinity to GITR.

In another preferred embodiment, the amino acid sequence of any of the above-mentioned CDRs includes a derivative CDR sequence with 1, 2 or 3 amino acids added, deleted, modified and/or substituted, and the derivative antibody comprising the VH and VL containing the derivative CDR sequence can retain the affinity of binding to GITR.

In another preferred embodiment, the ratio (F1/F0) of the binding affinity F1 between the derivatized antibody and GITR to the binding affinity F0 between the corresponding non-derivatized antibody and GITR is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to GITR, is an amino acid sequence having a homology or sequence identity of at least 96%.

In another preferred embodiment, the antibody further comprises a heavy chain constant region and/or a light chain constant region.

In another preferred embodiment, the heavy chain constant region is of human origin, and/or the light chain constant region is of human origin.

In another preferred embodiment, the heavy chain variable region of the antibody further comprises a human-derived framework region, and/or the light chain variable region of the antibody further comprises a human-derived framework region.

In another preferred embodiment, the heavy chain variable region of the antibody further comprises a murine-derived framework region, and/or the light chain variable region of the antibody further comprises a murine-derived framework region.

In another preferred embodiment, the antibody is selected from the group consisting of: animal-derived antibodies, chimeric antibodies, humanized antibodies, fully human antibodies, and a combination thereof.

In another preferred embodiment, the ratio (Z1/Z0) of the immunogenicity Z1 of the chimeric antibody in human to the immunogenicity Z0 of the non-chimeric antibody (such as murine-derived antibody) in human is 0-0.5, preferably 0-0.2, more preferably 0-0.05 (e.g. 0.001-0.05).

In another preferred embodiment, the antibody is a partially or fully humanized or fully human monoclonal antibody.

In another preferred embodiment, the antibody is a double chain antibody or a single chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody is in the form of a drug conjugate.

In another preferred embodiment, the antibody has one or more properties selected from the group consisting of:
(a) inhibiting tumor cell migration or metastasis;
(b) inhibiting tumor growth.

In another preferred embodiment, the antibody comprises the heavy chain variable region according to the first aspect of the present invention and the light chain variable region according to the third aspect of the present invention;
wherein, the heavy chain variable region and the light chain variable region comprise CDRs selected from the following group:

| VH-CDR1 | VH-CDR2 | VH-CDR3 | VL-CDR1 | VL-CDR2 | VL-CDR3 |
|---|---|---|---|---|---|
| Sequence number | Sequence number | Sequence number | Sequence number | Sequence number | Sequence number |
| 2 | 3 | 4, 43, 45 | 6 | 7 | 8 |
| 10 | 11 | 12 | 14 | 15 | 16 |
| 18 | 19 | 20 | 22 | 23 | 24 |
| 26 | 27 | 28 | 30 | 31 | 32 |

wherein, any one of the above amino acid sequences also includes a derivative sequence that is optionally with at least one amino acid added, deleted, modified, and/or substituted, and is capable of retaining the binding affinity to GITR.

In another preferred embodiment, the antibody comprises the heavy chain variable region according to the first aspect of the present invention and the light chain variable region according to the third aspect of the present invention; wherein,
the heavy chain variable region comprises the following three complementary determining regions or CDRs:
   VH-CDR1 shown in SEQ ID NO: 2,
   VH-CDR2 shown in SEQ ID NO: 3, and
   VH-CDR3 shown in SEQ ID NO: 4 or 43 or 45;
the light chain variable region comprises the following three complementary determining regions or CDRs:
   VL-CDR1 shown in SEQ ID NO: 6,
   VL-CDR2 shown in SEQ ID NO: 7, and
   VL-CDR3 shown in SEQ ID NO: 8;
      or
the heavy chain variable region comprises the following three complementary determining regions or CDRs:
   VH-CDR1 shown in SEQ ID NO: 10,
   VH-CDR2 shown in SEQ ID NO: 11, and
   VH-CDR3 shown in SEQ ID NO: 12;
the light chain variable region comprises the following three complementary determining regions or CDRs:
   VL-CDR1 shown in SEQ ID NO: 14,
   VL-CDR2 shown in SEQ ID NO: 15, and
   VL-CDR3 shown in SEQ ID NO: 16;
      or
the heavy chain variable region comprises the following three complementary determining regions or CDRs:
   VH-CDR1 shown in SEQ ID NO: 18,
   VH-CDR2 shown in SEQ ID NO: 19, and
   VH-CDR3 shown in SEQ ID NO: 20;
the light chain variable region comprises the following three complementary determining regions or CDRs:
   VL-CDR1 shown in SEQ ID NO: 22,
   VL-CDR2 shown in SEQ ID NO: 23, and
   VL-CDR3 shown in SEQ ID NO: 24;
      or
the heavy chain variable region comprises the following three complementary determining regions or CDRs:
   VH-CDR1 shown in SEQ ID NO: 26,
   VH-CDR2 shown in SEQ ID NO: 27, and
   VH-CDR3 shown in SEQ ID NO: 28;
the light chain variable region comprises the following three complementary determining regions or CDRs:
   VL-CDR1 shown in SEQ ID NO: 30,
   VL-CDR2 shown in SEQ ID NO: 31, and
   VL-CDR3 shown in SEQ ID NO: 32.

In another preferred embodiment, the heavy chain variable region of the antibody has the amino acid sequence shown in SEQ ID NO:1, 9, 17, 25, 41, 42, 44 or 46, and/or the light chain variable region of the antibody has the amino acid sequence shown in SEQ ID NO: 5, 13, 21, 29, 47, 48 or 49.

In another preferred embodiment, the heavy chain variable region of the antibody has the amino acid sequence shown in SEQ ID NO: 42, and the light chain variable region of the antibody has the amino acid sequence shown in SEQ ID NO: 5.

In another preferred embodiment, the heavy chain variable region of the antibody has the amino acid sequence shown in SEQ ID NO: 44, and the light chain variable region of the antibody has the amino acid sequence shown in SEQ ID NO: 5.

In another preferred embodiment, the antibody is selected from the group consisting of:

| Antibody number | Clone | VH sequence number | VL sequence number |
|---|---|---|---|
| 1 (3503-mAb019) | 96A10H9 | 1 | 5 |
| 2 (3503-mAb070) | 272G5E1 | 9 | 13 |
| 3 (3503-mAb076) | 277C12G4 | 17 | 21 |
| 4 (3503-mAb064) | 265G9A11 | 25 | 29 |
| 5 (3503-hab019e1) | 96A10H9 | 41 | 5 |
| 6 (3503-hab019e2) | 96A10H9 | 42 | 5 |
| 7 (3503-hab019e3) | 96A10H9 | 44 | 5 |
| 8 (3503-hab070e1) | 272G5E1 | 9 | 48 |
| 9 (3503-hab076e1) | 277C12G4 | 17 | 49 |
| 10 (3503-hab064e1) | 265G9A11 | 46 | 47. |

In another preferred embodiment, the amino acid sequence of the heavy chain variable region has a sequence homology or sequence identity of at least 80%, 85%, 90%, 91%, 92%, 93%, 94%, 95%, 96%, 97%, 98% or 99% with the amino acid sequence shown in SEQ ID NO: 1, 9, 17, 25, 41, 42, 44 or 46 in the sequence listing.

In another preferred embodiment, the amino acid sequence of the light chain variable region has a sequence homology or sequence identity of at least 80%, 85%, 90%, 91%, 92%, 93%, 94%, 95%, 96%, 97%, 98% or 99% with the amino acid sequence shown in SEQ ID NO: 5, 13, 21, 29, 47, 48 or 49 in the sequence listing.

In a sixth aspect of the present invention, it provides a recombinant protein, wherein the recombinant protein comprises:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(ii) an optional tag sequence that assists expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, a dimer, or a multimer.

In another preferred embodiment, the recombinant protein comprises:
(i) an antibody selected from the group consisting of,

| Antibody number | Clone | VH sequence number | VL sequence number |
|---|---|---|---|
| 1 (3503-mAb019) | 96A10H9 | 1 | 5 |
| 2 (3503-mAb070) | 272G5E1 | 9 | 13 |
| 3 (3503-mAb076) | 277C12G4 | 17 | 21 |
| 4 (3503-mAb064) | 265G9A11 | 25 | 29 |
| 5 (3503-hab019e1) | 96A10H9 | 41 | 5 |
| 6 (3503-hab019e2) | 96A10H9 | 42 | 5 |
| 7 (3503-hab019e3) | 96A10H9 | 44 | 5 |
| 8 (3503-hab070e1) | 272G5E1 | 9 | 48 |
| 9 (3503-hab076e1) | 277C12G4 | 17 | 49 |
| 10 (3503-hab064e1) | 265G9A11 | 46 | 47 |

and
(ii) an optional tag sequence to assist expression and/or purification.

In a seventh aspect of the present invention, it provides a polynucleotide, which encodes a polypeptide selected from the group consisting of:
(1) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(2) the recombinant protein according to the sixth aspect of the present invention.

In another preferred embodiment, the polynucleotide encoding the heavy chain variable region is shown in SEQ ID NO: 33, 35, 37, 39, 50, 51, 52 or 53; and/or the polynucleotide encoding the light chain variable region is shown in SEQ ID NO: 34, 36, 38, 40, 54, 55 or 56.

In another preferred embodiment, the polynucleotide encoding the heavy chain variable region sequence and the polynucleotide encoding the light chain variable region sequence are selected from the group consisting of:

| Clone | Sequence number of the polynucleotide encoding VH | Sequence number of the polynucleotide encoding VL |
|---|---|---|
| 1 (3503-mAb019) | 33 | 34 |
| 2 (3503-mAb070) | 35 | 36 |
| 3 (3503-mAb076) | 37 | 38 |
| 4 (3503-mAb064) | 39 | 40 |
| 5 (3503-hab019e1) | 50 | 34 |
| 6 (3503-hab019e2) | 51 | 34 |
| 7 (3503-hab019e3) | 52 | 34 |
| 8 (3503-hab070e1) | 35 | 55 |
| 9 (3503-hab076e1) | 37 | 56 |
| 10 (3503-hab064e1) | 53 | 54. |

In an eighth aspect of the present invention, it provides a vector, which contains the polynucleotide according to the seventh aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In a ninth aspect of the present invention, it provides a genetically engineered host cell, wherein the host cell contains the vector according to the eighth aspect of the present invention or the genome thereof is integrated with the polynucleotide according to the seventh aspect of the present invention.

In a tenth aspect of the present invention, it provides an antibody conjugate, which comprises:
(i) an antibody moiety, which is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, and a combination thereof; and
   (b) a coupling moiety coupled to the antibody moiety, and the coupling moiety is selected from the group consisting of a detectable label, a drug, a toxin, a cytokine, a radionuclide, an enzyme, and a combination thereof.

In another preferred embodiment, the antibody moiety is coupled to the coupling moiety via a chemical bond or linker.

In an eleventh aspect of the present invention, it provides an immune cell, which expresses or is exposed outside the cell membrane with the antibody according to the fifth aspect of the present invention.

In another preferred embodiment, the immune cell includes NK cells and T cells.

In another preferred embodiment, the immune cell is derived from human or non-human mammals (such as mice).

In a twelfth aspect of the present invention, it provides a pharmaceutical composition, wherein the pharmaceutical composition comprises:
(i) an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% of the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, and 0.01-99.99% of the pharmaceutically acceptable carrier, wherein the percentage is the mass percentage of the pharmaceutical composition.

In a thirteenth aspect of the present invention, it provides use of an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof, wherein the active ingredient is used for (a) preparation of a diagnostic reagent or kit; and/or (b) preparation of a medicine for preventing and/or treating diseases associated with abnormal GITR expression or function.

In another preferred embodiment, the diagnostic reagent is a detection piece or a detection plate.

In another preferred embodiment, the disease associated with abnormal GITR expression or function is selected from the group consisting of: cancer, tumor, and infectious diseases.

In another preferred embodiment, the diagnostic reagent or kit is used for:
(1) detection of GITR protein in a sample; and/or
(2) detection of endogenous GITR protein in tumor cells; and/or
(3) detection of tumor cells expressing GITR protein;
wherein the drug is used for preventing and/or treating diseases related to abnormal GITR expression or function, and the diseases related to abnormal GITR expression or function are cancer, tumor and infectious diseases.

In another preferred embodiment, the tumor is selected from the group consisting of melanoma, blastoma, lymphoma, hematoma, sarcoma, and adenoma.

In another preferred embodiment, the antibody is in the form of a drug conjugate (ADC).

In another preferred embodiment, the diagnostic reagent or kit is used for diagnosis of GITR related diseases.

In another preferred embodiment, the diagnostic reagent or kit is used for detection of GITR protein in a sample.

In a fourteenth aspect of the present invention, it provides a method for *in vitro* detection (including diagnostic or non-diagnostic) of GITR protein in a sample, wherein the method comprises the steps:
(1) contacting the sample with the antibody according to the fifth aspect of the present invention *in vitro;*
(2) detecting whether an antigen-antibody complex is formed, wherein the formation of the complex indicates the presence of GITR protein in the sample.

In a fifteenth aspect of the present invention, it provides a composition for detecting GITR protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, as an active ingredient.

In a sixteenth aspect of the present invention, it provides a detection plate, wherein the detection plate comprises: a substrate (support plate) and a detection strip, wherein the detection strip comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof.

In a seventeenth aspect of the present invention, it provides a kit, which comprises:
(1) a first container, which contains the antibody of the present invention;
   and/or
(2) a second container, which contains a secondary antibody against the antibody of the present invention;
   or,
the kit comprises the detection plate according to the sixteenth aspect of the present invention.

In an eighteenth aspect of the present invention, it provides a method for preparing a recombinant polypeptide, wherein the method comprises:
(a) culturing the host cell according to the ninth aspect of the present invention under conditions suitable for expression;
(b) isolating a recombinant polypeptide from the culture, wherein the recombinant polypeptide is the antibody according to the fifth aspect of the present invention or the recombinant protein according to the sixth aspect of the present invention.

In a nineteenth aspect of the present invention, it provides a drug combination, comprising:
(i) a first active ingredient, which comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof;
(ii) a second active ingredient, which comprises a second antibody, or a chemotherapeutic agent.

In another preferred embodiment, the second antibody is selected from the group consisting of a CTLA4 antibody, a PD-1 antibody, and a PD-L1 antibody.

In another preferred example, the chemotherapeutic agent is selected from the group consisting of docetaxel, carboplatin, and a combination thereof.

In a twentieth aspect of the present invention, it provides use of a combination for preparation of a medicine for the treatment of diseases associated with abnormal GITR expression or function, wherein the combination comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention, as well as a second antibody or a chemotherapeutic agent.

In another preferred embodiment, the second antibody is selected from the group consisting of a CTLA4 antibody, a PD-1 antibody, and a PD-L1 antibody.

In a twenty-first aspect of the present invention, it provides a method for the treatment of diseases associated with abnormal GITR expression or function, which comprises administering an effective amount of the antibody according to the fifth aspect of the present invention, the recombinant protein according to the sixth aspect of the present invention, the antibody conjugate according to the tenth aspect of the present invention, the immune cell according to the eleventh aspect of the present invention, the pharmaceutical composition of the twelfth aspect of the present invention, or a combination thereof, to a subject in need.

In another preferred embodiment, the disease associated with abnormal GITR expression or function is cancer, tumor, and an infectious disease.

In another preferred embodiment, the tumor is selected from the group consisting of melanoma, blastoma, lymphoma, hematoma, sarcoma, adenoma and the like.

In another preferred embodiment, the method further comprises: administering a safe and effective amount of a second antibody to the subject before, during, and/or after administering the first active ingredient.

In another preferred embodiment, the second antibody is selected from the group consisting of a PD-1 antibody and a CTLA4 antibody.

In another preferred embodiment, the second antibody is a PD-1 antibody.

It should be understood that within the scope of the present invention, the various technical features of the present invention above and the various technical features specifically described hereinafter (as in the embodiments) may be combined with each other to constitute a new or preferred technical solution. which needs not be described one by one, due to space limitations.

### Description of Drawings

FIG. 1 shows the results of FACS screening assays for 293F cells (A) and Renca cells (B) transfected with hGITR plasmid.
FIG. 2 shows the binding activity of fully human anti-GITR antibody to hGITR-ECD-hFc (A) and cGITR-ECD-hFc (B) determined by ELISA.
FIG. 3 shows the binding activity of fully human anti-GITR antibodies to 293F-hGITR (A) and 293F-cGITR (B) determined by FACS.
FIG. 4 shows the activity of fully human anti-GITR antibody to activate and bind NF-κB; (A) Non-crossing; (B) Crosslinking.
FIG. 5 shows the activity of fully human anti-GITR antibody to activate T cell.
FIG. 6 shows the binding activity of engineered fully human anti-GITR antibodies to hGITR-ECD-hFc (A) and cGITR-ECD-hFc (B) determined by ELISA.
FIG. 7 shows the binding activity of engineered fully human anti-GITR antibodies to 293F-hGITR (A) and 293F-cGITR (B) determined by FACS.
FIG. 8 shows the activity of engineered fully human anti-GITR antibody to activate NF-κB; (A) Non-crossing; (B) Crosslinking.
FIG. 9 shows the activity of engineered fully human anti-GITR antibody to activate T cell.
FIG. 10 shows the binding activity of engineered fully human anti-GITR antibody to GITR humanized mouse spleen cells.
FIG. 11 shows the weight change of mice after grouping.
FIG. 12 shows the changes in tumor volume of mice after grouping.

### Modes for carrying out the present invention

Through extensive and intensive studies, the inventor used three antigens of human GITR protein, human GITR gene, and human GITR expression cell line to immunize Harbour transgenic mouse H2L2 respectively, and harvested immune mouse spleen cells and fused them with myeloma cell line to obtain hybridioma cells. A group of human and mouse chimeric anti-human GITR antibodies with a new amino acid sequence were screened and produced. Then, the heavy chain variable region gene sequence of the preamble human GITR antibody was cloned into the expression vector containing signal peptide and heavy chain WT hIgG1 constant region by genetic engineering technology, and the light chain variable region gene sequence was cloned into the expression vector containing signal peptide and hIgG1 light chain κ constant region, and expressed and purified to obtain the parent fully human anti-GITR antibody (fully humanized anti-GITR antibody). Candidate fully human anti-GITR antibodies were screened by biological activity, physical and chemical activity and other aspects for further analysis and research. In the present invention, the variable region sequence of the fully human antibodies obtained by the rat-human chimeric antibody transgenic mouse (for example, 293F-hGITR cells and Renca-hGITR cells are used to immunize the transgenic mouse) is different from the existing antibodies. The experimental results show that: the fully human antibody of the present invention has a strong affinity; it has a good NF-κB activation activity; it has a good stimulating T cell activation activity; it has shown significant inhibition of tumor growth and improved mouse survival in mice. In addition, the fully human antibody of the present invention has better thermal stability. In addition, the invention prepared the engineering modified variant of the selected parent antibody through the genetic modification technology, and tested and identified the biological function and/or physical and chemical properties of the modified antibody, selected candidate fully human anti-GITR variant antibodies, and further evaluated the biological activity, physicochemical properties and other aspects of the candidate antibodies. The present invention has been completed on the basis of this.

### Terms

In the present invention, "VH" refers to the heavy chain variable region and "VL" refers to the light chain variable region. "VH-CDR1" refers to CDR1 of the heavy chain variable region; "VH-CDR2" refer to CDR2 of the heavy chain variable region; "VH-CDR3" refer to CDR3 of the heavy chain variable region. "VL-CDR1" refer to CDR1 of the light chain variable region; "VL-CDR2" refer to CDR2 of the light chain variable region; "VL-CDR3" refer to CDR3 of the light chain variable region.

### GITR

GITR (Glucocorticoid-induced TNFR-related Protein) is a gene associated with glucocorticoid-induced TNFR, which belongs to the TNF receptor family. GITR is a type I transmembrane protein, and its extracellular domain is rich in cysteine residues, which is a common feature of TNFR family members.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). The variable regions of the natural heavy and light chains each contain four FR regions, which are roughly in the β-folded configuration, connected by the three CDRs that form the connecting loop, and in some cases may form a partly β folded structure. The CDRs in each chain are closely linked together via the FR regions, and together with the CDRs of the other chain, form the antigen binding site of an antibody (see Kabat *et al.,* NIH Publ. No. 91-3242, Vol. I, pp. 647-669 (1991)). The constant regions are not directly involved in the binding of an antibody to an antigen, however, they exhibit different effector functions, such as involved in the antibody-dependent cytotoxicities of an antibody.

The light chains of vertebrate antibodies (immunoglobulins) can be classified into one of two distinct classes (referred to as κ and λ) based on the amino acid sequence of their constant regions. Immunoglobulins can be classified into different classes depending on the amino acid sequences of their heavy chain constant regions. There are mainly five classes of immunoglobulins: IgA, IgD, IgE, IgG, and IgM, some of which can be further classified into subclasses (isotypes), such as IgG1, IgG2, IgG3, IgG4, IgA, and IgA2. The heavy chain constant regions corresponding to different classes of immunoglobulins are called α, δ, ε, γ, and µ, respectively. The subunit structures and three-dimensional configurations of different classes of immunoglobulins are well known for those skilled in the art.

In general, the antigen binding characteristics of an antibody can be described by three specific regions located in the heavy and light chain variable regions, called complementarity determining regions (CDRs), which divide the variable region into four framework regions (FRs); the amino acid sequences of the four FRs are relatively conservative and are not directly involved in the binding reaction. These CDRs form a ring structure, and approach to each other in the steric structure by virtue of the β-sheets formed by the FRs between them, and the CDRs on the heavy chain and the CDRs on the corresponding light chain constitute the antigen-binding site of an antibody. By comparison of the amino acid sequences of antibodies of the same type, it can be determined which amino acids form FRs or CDRs.

The present invention includes not only an intact antibody, but also the fragments of the antibody having an immunological activity or a fusion protein formed by the antibody and another sequence. Therefore, the present invention also includes fragments, derivatives and analogs of the antibody.

In the present invention, antibodies include murine, chimeric, humanized or fully humanized antibodies as prepared by techniques well known to those skilled in the art. Recombinant antibodies, such as chimeric and humanized monoclonal antibodies, including human and non-human portions, can be obtained by standard DNA recombination techniques, all of which are useful antibodies. A chimeric antibody is a molecule in which different portions are derived from different animal species, for example, a chimeric antibody having a variable region from a monoclonal antibody from a mouse and a constant region from a human immunoglobulin (see, for example, U.S. Pat. Nos. 4,816,567 and 4,816,397, which are incorporated herein by reference in its entirety). A humanized antibody refers to an antibody molecule derived from a non-human species, which has one or more complementarity determining regions (CDRs) derived from a non-human species and framework regions derived from a human immunoglobulin molecule (see U.S. Pat. No. 5,585,089, which is incorporated herein by reference in its entirety). These chimeric and humanized monoclonal antibodies can be prepared by recombinant DNA techniques well known in the art.

In the present invention, an antibody may be monospecific, bispecific, trispecific, or multispecific.

In the present invention, the antibody of the present invention further includes a conservative variant thereof, which refers to a polypeptide formed by substitution of at most 10, preferably at most 8, more preferably at most 5, and most preferably at most 3 amino acids with amino acids having similar or analogous property, as compared to the amino acid sequence of the antibody of the present invention. These conservatively variant polypeptides are preferably produced by amino acid substitution according to Table 25.

**Table 25**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-GITR antibody

In the present invention, the antibody is an anti-GITR antibody. The present invention provides an antibody with high specificity and high affinity against GITR, which comprises a heavy chain and a light chain, wherein the heavy chain contains a heavy chain variable region (VH) amino acid sequence, and the light chain contains a light chain variable region (VL) amino acid sequence.

Preferably,
the heavy chain variable region (VH) has a complementarity determining region or CDR selected from the group consisting of:
   VH-CDR1 shown in SEQ ID NO: 8n+2,
   VH-CDR2 shown in SEQ ID NO: 8n+3, and
   VH-CDR3 shown in SEQ ID NO: 8n+4;
   wherein, each n is independently 0, 1, 2 or 3;
the light chain variable region (VL) has a complementarity determining region or CDR selected from the group consisting of:
   VL-CDR1 shown in SEQ ID NO: 8n+6,
   VL-CDR2 shown in SEQ ID NO: 8n+7, and
   VL-CDR3 shown in SEQ ID NO: 8n+8;
   wherein, each n is independently 0, 1, 2 or 3;
   wherein, any one of the above amino acid sequences also includes a derivative sequence that is optionally with at least one amino acid added, deleted, modified, and/or substituted, and is capable of retaining the binding affinity to GITR.

Preferably, the heavy chain variable region (VH) comprises the following three complementary determining regions or CDRs:
VH-CDR1 shown in SEQ ID NO: 8n+2,
VH-CDR2 shown in SEQ ID NO: 8n+3, and
VH-CDR3 shown in SEQ ID NO: 8n+4;
the light chain variable region (VL) comprises the following three complementary determining regions or CDRs:
VL-CDR1 shown in SEQ ID NO: 8n+6,
VL-CDR2 shown in SEQ ID NO: 8n+7, and
VL-CDR3 shown in SEQ ID NO: 8n+8;
wherein, each n is independently 0, 1, 2 or 3; preferably n is 0;
wherein, any one of the above amino acid sequences also includes a derivative sequence that is optionally with at least one amino acid added, deleted, modified, and/or substituted, and is capable of retaining the binding affinity to GITR.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Methods known to those of ordinary skill in the art for determining sequence homology or identity include, but are not limited to: Computational Molecular Biology, Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., Stockton Press, New York, 1991, and Carillo, H. and Lipman, D., SIAM J. Applied Math., 48:1073 (1988). The preferred method for determining identity is to obtain the greatest match between the sequences tested. Methods for determining identity are compiled into publicly available computer programs. Preferred computer program method for determining identity between two sequences includes, but are not limited to, the GCG software package (Devereux, J. et al., 1984), BLASTP, BLASTN, and FASTA (Altschul, S, F. et al., 1990). The BLASTX program is available to the public from NCBI and other sources (BLAST Handbook, Altschul, S. et al., NCBI NLM NIH Bethesda, Md. 20894; Altschul, S. et al., 1990). The well-known Smith Waterman algorithm can also be used to determine identity.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

The antibody derivative of the present invention may be a single-chain antibody, and/or an antibody fragment, for example, Fab, Fab', (Fab')2 or other antibody derivatives known in the art, etc., and may be any one or more of IgA, IgD, IgE, IgG and IgM antibodies or other subtype antibodies.

In the present invention, the animal is preferably a mammal, such as a mouse.

The antibody of the present invention may be a chimeric antibody, a humanized antibody, a CDR grafted and/or modified antibody targeting GITR (such as human GITR).

In above content of the present invention, the number of the added, deleted, modified and/or substituted amino acids, preferably does not exceed 40%, more preferably does not exceed 35%, more preferably is 1-33%, more preferably is 5-30%, more preferably is 10-25%, and more preferably is 15-20% of the total number of the amino acids of the initial amino acid sequence.

In the above content of the present invention, more preferably, the number of the added, deleted, modified and/or substituted amino acids, may be 1-7, more preferably 1-5, more preferably 1-3, and more preferably 1-2.

In another preferred embodiment, the heavy chain variable region of the antibody has the amino acid sequence shown in SEQ ID NO: 1, 9, 17, 25, 41, 42, 44 or 46.

In another preferred embodiment, the light chain variable region of the antibody has the amino acid sequence shown in SEQ ID NO: 5, 13, 21, 29, 47, 48 or 49.

In another preferred embodiment, the amino acid sequences of the heavy chain variable region and/or the light chain variable region of the antibody targeting GITR are shown in the following Table 26:

**Table 26**

| | | |
|---|---|---|
| Antibody number | VH sequence number (SEQ ID No.) | VL sequence number (SEQ ID No.) |
| 1 | 1 | 5 |
| 2 | 9 | 13 |
| 3 | 17 | 21 |
| 4 | 25 | 29 |
| 5 | 41 | 5 |
| 6 | 42 | 5 |
| 7 | 44 | 5 |
| 8 | 9 | 48 |
| 9 | 17 | 49 |
| 10 | 46 | 47 |

In another preferred embodiment, the antibodies targeting GITR are 3503-mAb019, 3503-mAb070, 3503-mAb076, 3503-mAb064, 3503-hab019e1, 3503-hab019e2, 3503-hab019e3, 3503-hab070e1, 3503-hab076e1, and 3503-hab064e1.

### Engineering Modification of fully human Anti-GITR Antibody

In the invention, the first step is to obtain a human-mouse chimeric antibody through immunization; the second step is to replace the Fc fragment with human Fc to obtain a fully human antibody; and the third step is to change 1-3 bases through engineering modification to remove hotspot in the variable region or to perform reversal mutation and other related operations after comparing with Germline sequence to obtain an engineering modified fully human antibody, which is called "engineering modified fully human anti-GITR antibody" or "engineering modified fully humanized anti-GITR antibody".

### Recombinant protein

The invention also provides a recombinant protein comprising one or more of heavy chain CDR1 (VH-CDR1), heavy chain CDR2 (VH-CDR2) and heavy chain CDR3 (VH-CDR3) of the GITR antibody, and/or one or more of light chain CDR1 (VL-CDR1), light chain CDR2 (VL-CDR2) and light chain CDR3 (VL-CDR3) of the GITR antibody,

The sequences of the heavy chain CDR1-3 are as follows:
VH-CDR1 shown in SEQ ID NO: 8n+2,
VH-CDR2 shown in SEQ ID NO: 8n+3, and
VH-CDR3 shown in SEQ ID NO: 8n+4;
the sequences of the light chain CDR1-3 are as follows:
   VL-CDR1 shown in SEQ ID NO: 8n+6,
   VL-CDR2 shown in SEQ ID NO: 8n+7, and
   VL-CDR3 shown in SEQ ID NO: 8n+8;
   wherein, each n is independently 0, 1, 2 or 3; preferably n is 0;
   wherein, any one of the above amino acid sequences also includes a derivative sequence that is optionally with at least one amino acid added, deleted, modified, and/or substituted, and is capable of retaining the binding affinity of GITR.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

In another preferred embodiment, the recombinant protein of the present invention comprises a heavy chain variable region of a GITR antibody and/or a light chain variable region of a GITR antibody, and the heavy chain variable region of the antibody has the amino acid sequence shown in SEQ ID NO: 1, 9, 17, 25, 41, 42, 44 or 46; the light chain variable region of the antibody has the amino acid sequence shown in SEQ ID NO: 5, 13, 21, 29, 47, 48 or 49.

In another preferred embodiment, the recombinant protein of the present invention comprises a heavy chain variable region of a GITR antibody comprising an amino acid sequence shown in SEQ ID NO: 1, 9, 17, 25, 41, 42, 44 or 46 and a light chain variable region of a GITR antibody comprising an amino acid sequence shown in SEQ ID NO: 5, 13, 21, 29, 47, 48 or 49.

In another preferred embodiment, the amino acid sequence numbers of the recombinant protein and the heavy chain CDR1-3 and light chain CDR1-3 comprised therein are as shown in Table 27:

**Table 27 Amino acid sequence numbers of heavy chain CDR1-3 and light chain CDR1-3 (SEQ ID No.)**

| Isolated protein Number | VH-CDR1 SEQ ID No. | VH-CDR2 SEQ ID No. | VH-CDR3 SEQ ID No. | VL-CDR1 SEQ ID No. | VL-CDR2 SEQ ID No. | VL-CDR3 SEQ ID No. |
|---|---|---|---|---|---|---|
| 1 | 2 | 3 | 4 | 6 | 7 | 8 |
| 2 | 10 | 11 | 12 | 14 | 15 | 16 |
| 3 | 18 | 19 | 20 | 22 | 23 | 24 |
| 4 | 26 | 27 | 28 | 30 | 31 | 32 |
| 5 | 2 | 3 | 4 | 6 | 7 | 8 |
| 6 | 2 | 3 | 43 | 6 | 7 | 8 |
| 7 | 2 | 3 | 45 | 6 | 7 | 8 |
| 8 | 10 | 11 | 12 | 14 | 15 | 16 |
| 9 | 18 | 19 | 20 | 22 | 23 | 24 |
| 10 | 26 | 27 | 28 | 30 | 31 | 32 |

wherein, any one of the above amino acid sequences also includes a derivative sequence that is optionally with at least one amino acid added, deleted, modified, and/or substituted, and is capable of retaining the binding affinity to GITR.

Preferably, the recombinant protein also comprises an antibody heavy chain constant region and/or an antibody light chain constant region, wherein the antibody heavy chain constant region is conventional in the art, preferably a rat antibody heavy chain constant region or a human antibody heavy chain constant region, more preferably a human antibody heavy chain constant region. The antibody light chain constant region is conventional in the art, preferably a rat antibody light chain constant region or a human antibody light chain constant region, more preferably a human antibody light chain constant region.

The recombinant protein is a conventional protein in the art. Preferably, it is one or more of an antibody full-length protein, an antigen-antibody binding domain protein fragment, a bispecific antibody, a multispecific antibody, a single chain antibody fragment (scFv), a single domain antibody (sdAb) and a single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully humanized antibody full-length protein. Preferably, the antibody full-length protein is IgG1, IgG2, IgG3 or IgG4.

The single-chain antibody is a conventional single-chain antibody in the art, which comprises a heavy chain variable region, a light chain variable region and a short peptide of 15-20 amino acids.

The antigen-antibody binding domain protein fragments are conventional antigen-antibody binding domain protein fragments in the art, which comprise a light chain variable region, a light chain constant region, and an Fd segment of heavy chain constant region. Preferably, the antigen-antibody binding domain protein fragments are Fab and F (ab').

The single domain antibody is a conventional single domain antibody in the art, which comprises a heavy chain variable region and a heavy chain constant region.

The single-domain antibody is a conventional single-domain antibody in the art, which only comprises a heavy chain variable region.

Wherein, the preparation method of the recombinant protein is a conventional preparation method in the art. Preferably, the preparation method is: isolating and obtaining the protein from an expression transformant that recombinantly expresses the protein or obtaining the protein by artificially synthesizing a protein sequence. The method of isolating and obtaining the protein from an expression transformant that recombinantly expresses the protein is preferably as follows: cloning a nucleic acid molecule encoding the protein carrying a point mutation into a recombinant vector, and transforming the obtained recombinant vector into a transformant to obtain a recombinant expression transformant, and by culturing the obtained recombinant expression transformant, the recombinant protein can be obtained by separation and purification.

### Nucleic Acid

The present invention also provides a nucleic acid, which encodes the above-mentioned antibody (e.g., an anti-GITR antibody) or recombinant protein or the heavy chain variable region or the light chain variable region of the anti-GITR antibody.

Preferably, the nucleotide sequence of the nucleic acid encoding the heavy chain variable region is shown in SEQ ID NO: 33, 35, 37, 39, 50, 51, 52 or 53; and/or, the nucleotide sequence of the nucleic acid encoding the light chain variable region is shown in SEQ ID NO: 34, 36, 38, 40, 54, 55 or 56.

More preferably, the nucleotide sequence of the nucleic acid encoding the heavy chain variable region is shown in SEQ ID NO: 33, 35, 37, 39, 50, 51, 52 or 53, and the nucleotide sequence of the nucleic acid encoding the light chain variable region is shown in SEQ ID NO: 34, 36, 38, 40, 54, 55 or 56.

The preparation method of the nucleic acid is a conventional preparation method in the art. Preferably, it comprises the following steps: obtaining the nucleic acid molecule encoding the above-mentioned protein by gene cloning technology, or obtaining the nucleic acid molecule encoding the above-mentioned protein by the method of artificial full-length sequence synthesis.

Those skilled in the art know that the base sequence encoding the amino acid sequence of the protein can be replaced, deleted, changed, inserted or added appropriately to provide a polynucleotide homolog. The homolog of the polynucleotide of the present invention can be prepared by replacing, deleting or adding one or more bases of the gene encoding the protein sequence within the scope of maintaining the activity of the antibody.

### Vector

The present invention also provides a recombinant expression vector comprising the nucleic acid.

The recombinant expression vector can be obtained by conventional methods in the art, that is, by connecting the nucleic acid molecule of the present invention to various expression vectors, thus being constructed. The expression vector is one of a variety of conventional vectors in the art, as long as it can carry the above-mentioned nucleic acid molecule. The vector preferably includes: various plasmids, cosmids, phage or virus vectors and the like.

The present invention also provides a recombinant expression transformant comprising the above-mentioned recombinant expression vector.

Wherein, the preparation method of the recombinant expression transformant is a conventional preparation method in the art, preferably comprising: being obtained by transforming the recombinant expression vector into a host cell. The host cell is one of a variety of conventional host cells in the art, as long as the recombinant expression vector can replicate itself stably and the nucleic acid carried can be effectively expressed. Preferably, the host cell is *E.coli* TG1 or *E.coli* BL21 cell (for expressing single-chain antibodies or Fab antibodies), or HEK293 or CHO cell (for expressing full-length IgG antibodies). The above-mentioned recombinant expression plasmid is transformed into a host cell to obtain the preferred recombinant expression transformant of the present invention. The transformation method is a conventional transformation method in the art, preferably a chemical transformation method, a heat shock method or an electrotransformation method.

### Antibody preparation

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, supercentrifugation, molecular sieve chromatography (gel chromatography), adsorption chromatography, ion exchange chromatography, high performance liquid chromatography (HPLC), and any other liquid chromatography, and the combination thereof.

### Antibody-drug conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 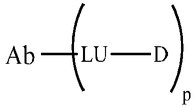 wherein:
Ab is an antibody,
LU is a linker;
D is a drug;
And the subscript p is a value selected from 1 to 8.

### Application

The present invention also provides use of the antibody, the antibody conjugate ADC, the recombinant protein, and/or immune cell of the present invention, for example for the preparation of diagnostic preparations or the preparation of drugs.

Preferably, the drug is for prevention and/or treatment of diseases associated with abnormal GITR expression or function.

In the present invention, the diseases associated with abnormal GITR expression or function are conventional diseases associated with abnormal GITR expression or function in the art. Preferably, the disease associated with abnormal GITR expression or function is cancer or tumor.

In the present invention, the cancer is a conventional cancer in the art, preferably colon cancer or breast cancer. In the present invention, the tumor disease is a conventional tumor disease in the art, preferably melanoma.

Uses of the antibody, the ADC, the recombinant protein, and/or the immune cell of the present invention include (but are not limited to):
(i) for diagnose, prevention and/or treatment of tumorigenesis, tumor growth and/or metastasis. The tumor includes (but is not limited to): hematoma, melanoma, sarcoma, lymphoma, adenoma, and blastoma.
(ii) for diagnosis, prevention and/or treatment of cancer, including (but not limited to): head and neck cancer, small cell lung cancer, non-small cell lung cancer, lung cancer, squamous cell cancer, colon cancer, colorectal cancer, renal cell cancer, gastrointestinal cancer, pancreatic cancer, cervical cancer, ovarian cancer, liver cancer, bladder cancer, testicular cancer, breast cancer, endometrial cancer, salivary gland cancer, prostate cancer, vulvar cancer, esophageal cancer, and thyroid cancer.
(iii) for diagnosis, prevention and/or treatment of tumor, including (but not limited to): melanoma, Hodgkin's and non-Hodgkin's lymphoma, glioblastoma, hepatocellular tumor, glioma, myeloma, Wilms' tumor, and hematoma.

### Use for detection and kit

The antibody or ADC thereof of the present invention can be used for detection, for example, for detecting samples, thereby providing diagnostic information.

In the present invention, the samples (specimens) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cell or tissue samples.

The present invention also provides a kit comprising the antibody (or fragment thereof) of the present invention. In a preferred embodiment of the present invention, the kit further includes a container, an instruction for use, buffer, and the like. In a preferred embodiment, the antibody of the present invention can be immobilized on a detection plate.

### Pharmaceutical composition

The invention further provides a composition. In the preferred examples, the composition is a pharmaceutical composition comprising the antibody, or an active fragment, a fusion protein or an ADC thereof, or a corresponding immune cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is in one of a variety of conventional dosage forms in the art, preferably in solid, semi-solid or liquid form, and can be an aqueous solution, a non-aqueous solution or a suspension, and more preferably tablets, capsules, granules, injection or infusion, etc.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for prevention and/or treatment of diseases associated with abnormal GITR expression or function.

The pharmaceutical composition according to the present invention can be directly used for binding to a GITR protein molecule, and thus can be used for preventing and treating diseases such as tumors.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 5 milligrams per kilogram body weight per day. Further, the polypeptide of the present invention can also be used in combination with the other therapeutic agents.

In the present invention, preferably, the pharmaceutical composition of the present invention further comprises one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipient is a conventional pharmaceutical excipient in the art, and preferably includes pharmaceutically acceptable excipients, fillers or diluents. More preferably, the pharmaceutical composition comprises 0.01-99.99% of the above-mentioned protein and 0.01-99.99% of the pharmaceutically acceptable carrier, wherein the percentage is the mass percentage of the pharmaceutical composition.

In the present invention, preferably, the administration amount of the pharmaceutical composition is an effective amount, and the effective amount is an amount that can alleviate or delay the progression of the disease, and the degenerative or traumatic condition. The effective amount can be determined on an individual basis and will be partly based on consideration of the symptoms to be treated and the results sought. Those skilled in the art can determine the effective amount by using the above-mentioned factors such as individual basis and using no more than conventional experiments.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 micrograms/kg body weight to about 20 mg/kg body weight. Of course, the particular dose should also depend on various factors, such as the route of administration, patient healthy status, which are well within the skills of an experienced physician.

The present invention provides use of the above-mentioned pharmaceutical composition in the preparation of a medicine for preventing and/or treating diseases associated with abnormal GITR expression or function. Preferably, the disease associated with abnormal GITR expression or function is cancer or tumor.

### Method and composition for detecting GITR protein in a sample

The present invention also provides a method for detecting GITR protein in a sample (for example, detecting cells over-expressing GITR), which comprises the following steps: contacting the above-mentioned antibody with a sample to be tested *in vitro,* and detecting whether the above-mentioned antibody binds to the sample to be tested, to form an antigen-antibody complex.

The meaning of overexpression is conventional in the art, which refers to the overexpression of RNA or protein of GITR protein in the sample to be tested (due to increased transcription, post-transcriptional processing, translation, post-translational processing and protein degradation changes), and local overexpression and increased functional activity (such as in the case of increased enzymatic hydrolysis of the substrate) due to changes in protein transport mode (increased nuclear localization).

In the present invention, the detection method for detecting whether an antigen-antibody complex is formed is a conventional detection method in the art, preferably a flow cytometry (FACS) detection.

The present invention provides a composition for detecting GITR protein in a sample, which comprises the above-mentioned antibody, recombinant protein, antibody conjugate, immune cell, or a combination thereof as an active ingredient. Preferably, it also comprises a compound composed of the functional fragments of the above-mentioned antibody as an active ingredient.

On the basis of conforming to common knowledge in the art, the above-mentioned preferred conditions can be combined arbitrarily to obtain preferred embodiments of the present invention.

The main advantages of the invention are:
(1) The transgenic mice used in the present invention can obtain fully human antibodies more easily than wild-type mice, therefore the immunogenicity of antibodies are reduced. And compared with transgenic mice of fully human antibodies, the number of antibodies obtained is larger, and the antibodies have strong affinity, good sequence diversity and high activity.
(2) Compared with antibodies obtained from phage library, the antibody of the present invention obtained by hybridoma technology has high affinity and good sequence expression.
(3) The present invention uses recombinantly expressed 293F-hGITR and Renca-hGITR cells. Compared with protein or polypeptide immunogens, the expressed target protein has a more natural conformation; and compared with other recombinantly expressed cells, its expression level is higher.
(4) The present invention obtains antibodies with different sequences, which can specifically bind to hGITR-ECD-hFc protein and cGITR-ECD-hFc protein, and can specifically bind to 293F stable cell lines expressing hGITR (293F-hGITR) and 293F stable cell lines expressing cGITR (293F-cGITR).
(5) The antibody of the present invention has the activity of activating NF-κB. By activating T cells, the secretion level of INF-γ is improved, and tumor growth can be inhibited *in vivo* and the survival rate of mice can be improved in human GITR transgenic mice. All activities are better than or equal to thoes antibodies from comparative documents.

The invention is further illustrated by the following specific examples. It is to be understood that these examples are for illustrative purposes only and are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Percentages and parts are by weight unless otherwise stated. The experimental materials and reagents used in the following examples are commercially available unless otherwise specified.

### EXAMPLE 1 Preparation of chimeric anti-GITR antibody

### (1) Preparation of immunogen A

The amino acid sequence Gln26-Glu161, Thr45Ala of the extracellular region of human GITR protein (as shown in SEQ ID No: 57 in the sequence listing) was cloned into the pCpC vector with human IgG Fc fragment (hFc) (purchased from Invitrogen, V044-50) and plasmids were prepared according to the established standard molecular biology methods. For specific methods, see Sambrook, J., Fritsch, E.F., and Maniatis T. (1989). Molecular Cloning: A Laboratory Manual, Second Edition (Plainview, New York: Cold Spring Harbor Laboratory Press). HEK293 cells (purchased from Invitrogen) were transiently transfected (PEI, Polysciences) and FreeStyle™293 (purchase from Invitrogen) was used at 37°C. After 4 days, the cell culture was collected, and the cell components were removed by centrifugation to obtain the culture supernatant containing the extracellular region of GITR protein. The culture supernatant was loaded onto a protein A affinity chromatography column (Mabselect Sure, purchased from GE Healthcare), and an ultraviolet (UV) detector was used to monitor the change in ultraviolet absorbance (A280). After the sample was loaded, the Protein A affinity chromatography column was washed with PBS phosphate buffer (pH 7.2) until the A280 value returned to baseline, and then eluted with 0.1 M glycine (pH 2.5) to obtain the human GITR extracellular domain protein with hFc tag (hGITR-ECD-hFc). Dialysis was performed overnight with PBS phosphate buffer (pH 7.2) at 4 °C. The dialyzed protein was filtered by a sterile filter with a pore size of 0.22 µm to obtain purified immunogen A. Immunogen A needed a series of quality control tests before use, such as tests of protein concentration, purity, molecular weight and ligand binding activity, etc.

### (2) Preparation of immunogen B

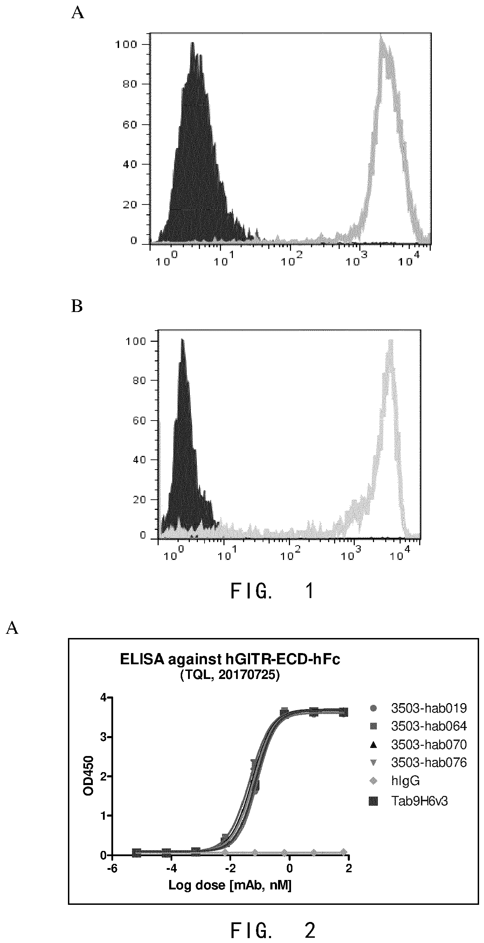

The nucleotide sequence encoding the full-length human GITR (the sequence is shown in SEQ ID No: 58 in the sequence listing) was cloned into the pIRES vector (purchased from Clontech), and the plasmid was prepared according to the above method. After plasmid transfection on 293F cell line and Renca cell line (both purchased from Invitrogen) (transfection was preformed using X-treme GENE HP DNA Transfection Reagent, which was purchased from Roche, Cat #06 366 236 001, and operated according to the instructions), cells were selectively cultured in DMEM medium containing 10% (w/w) FBS and containing 0.5 µg/ml puromycin for 2 weeks. Subcloning was conduted in 96-well culture plate by a limiting dilution method, and the plate was placed at 37°C, 5% (v/v) CO₂. After about 2 weeks, some of the monoclonal wells were selected and amplified into 6-well plates. The amplified clones were stained with known GITR antibodies and screened by flow cytometry. The culture expanding of the monoclonal cell line with better growth, higher fluorescence intensity was continued and cryopreserved in liquid nitrogen, thus obtaing immunogen B. The specific selection results are shown in Table 1 and FIG. 1. In Table 1, positive cells (%) refer to the percentage of number of positive cells in the total number of cells. FIG. 1 shows that 293F cells (clone number) and Renca cells (clone number) have higher levels of expression of hGITR.

**Table 1 FACS screening and detection results of 293F cells and Renca cells transfected with hGITR plasmid**

| Number | Mother cell | Recombinant cell clone ID | IgG control | | Anti-GITR antibody | |
|---|---|---|---|---|---|---|
| | | | MFI | Proportion of positive cells (%) | MFI | Proportion of positive cells (%) |
| 1 | 293F-hGITR | 4F9 | 3.5 | 3.6 | 1099.8 | 99.3 |
| 2 | | 4G8 | 3.1 | 1.8 | 584.2 | 99.6 |
| 3 | | 5F9 | 4.5 | 4.5 | 1107.8 | 99.6 |
| 4 | | 3B4 | 3.4 | 2.7 | 29.8 | 86.1 |
| 5 | | 4C11 | 4.7 | 8.2 | 1798.5 | 98.8 |
| 6 | Renca-hGITR | 4B7 | 2.8 | 0.2 | 672.0 | 88.9 |
| 7 | | 4B11 | 5.1 | 9.9 | 1074.0 | 95.8 |
| 8 | | 4G7 | 2.2 | 0.1 | 1166.0 | 95.6 |
| 9 | | 5C3 | 2.5 | 0.1 | 1024.0 | 96.0 |
| 10 | | 5F2 | 2.7 | 0.4 | 1137.0 | 90.9 |

The above results show that the stable cell lines 293F-hKLB and Renca-hKLB with high expression of human GITR gene are obtained in the present invention, which can be used for immunogen and/or antibody screening and functional identification.

### (3) Preparation of immunogen C

The full-length nucleotide sequence encoding human GITR molecule (as shown in SEQ ID No. 58 in sequence listing) was cloned into a pCpC vector and the plasmid was prepared.

### (4) Preparation of hybridoma cells and screening of antibody

Harbour transgenic mice are introduced with human immunoglobulin variable region genes and rat immunoglobulin constant region genes, while the Ig expression of the mice own is silenced (F.G. Franklin, et al, patent #WO 2010/070263 A1). The transgenic mice can produce immune response and antibody titer equivalent to that produced by normal mice (such as Balb/c) after being immunized with antigen.

A. 6-8 weeks old Harbour H2L2 human antibody transgenic mice (purchased from Beijing Weitong Lihua Company) were used for immunization by immunogen A, and the mice were raised under SPF conditions. During the first immunization, 50 g of immunogen A was injected into the abdominal cavity of each mouse together with 250 µl of complete Freund's adjuvant (CFA). After the first immunization, the first boosting immunization was performed at intervals of 2 weeks. 50 g of immunogen A was injected into the abdominal cavity of each mouse together with 250 µl of incomplete Freund's adjuvant (IFA), and then boosting immunization was performed again at intervals of 3 weeks. Blood samples were collected one week after each boosting immunization, and the antibody titer and specificity of immunogen A in serum were detected by FACS.

B. 6-8 weeks old Harbour H2L2 human antibody transgenic mice (purchased from Beijing Weitong Lihua Company) were used for immunization by immunogen B, and the mice were raised under SPF conditions. Stably transfected 293F-hGITR cells (clone number: 4C11) or Renca-hGITR cells (clone number: 5F2) were inoculated into T-75 flask at 2×10⁶ cell density. The cells were cultured in DMEM medium containing 10% FBS and 0.5 µg/ml puromycin. When the cells reach 90% confluence, the medium was sucked out, and the cells were washed twice with DMEM medium, and treated with non-enzymatic cell dissociation buffer at 37 °C until the cells were separated from the culture flask. The cells were collected and washed twice with DMEM medium, and the number of cells was determined and adjusted to 1×10⁸ cells/mL using PBS buffer (pH 7.2), treated with mitomycin at a final concentration of 25 ug/ml for 3 hours, centrifuged and washed with PBS, the number of cells were measured and adjusted to 1×10⁷ cells/mL using PBS buffer (pH 7.2). Each mouse was immunized with 500 µl cell suspension. Two weeks after the first immunization, the first boosting immunization was performed, and then boosting immunization was performed at intervals of 3 weeks. Blood samples were collected one week after immunization. The titer and specificity of antibody in serum were determined by FACS.

C. 6-8 weeks old Harbour H2L2 human antibody transgenic mice (purchased from Beijing Weitong Lihua Company) were used for immunization by immunogen C, and the mice were raised under SPF conditions. All mice were immunized with Helios gene gun for 4 times, and each immunization was injected 4 times. Each injection contained 1 µg of DNA. The first boosting immunization was given at intervals of 2 weeks after the first immunization. After that, the boosting immunization was given at intervals of 3 weeks. Blood samples were collected one week after immunization, and antibody titers and specificity in serum were determined by FACS.

Before performing cell fusion, mice having a better immune response to hGITR were selected to boosting immunization with 100 µg of purified hGITR-ECD-hFc via IP pathway (for mice immunized with protein immunogen or gene immunogen), or to boosting immunization via IP pathway with 500 µl (5 × 10⁶ cells) 293F-hGITR or Renca-hGITR for cell immunization group, to preform a final boosting immunization. After 3-5 days, the mice were sacrificed by euthanasia and splenocytes were collected. Adding NH₄OH to the final concentration of 1%, red blood cells in spleen cell suspension were washed with DMEM basic medium by centrifugation for 2-3 times, and then mixed with mouse myeloma cells SP2/0 at a ratio of 5: 1. The traditional PEG cell fusion method or high-efficiency electrofusion method was used for cell fusion. The fused cells were diluted into DMEM selective medium containing 20% fetal bovine serum and 1xHAT, and were added at 1 × 10⁵/20µL per well to a 96-well cell culture plate and placed in a 5% CO₂, 37 °C incubator. After 10-14 days, the supernatant of cell fusion plate was screened by FACS (flow cytometry) or NF-κB reporter gene experiment, and the positive clones were amplified to 24-well plate for expansion culture. After 2-3 days, the 24-well plate supernatant was analyzed for antibody subtypes. The binding activity to GITR protein and GITR positive cells was determined by ELISA and FACS, the activation activity to NF-κB reporter gene was determined by fluorescein detection, and the activation activity to T cells was determined by INF-γ detection.

According to the screening results of 24-well plate, the desired clones were selected and subcloned on 96-well plate by limiting dilution analysis. 7-10 days after subcloning, FACS and NF-κB reporter gene experiments were used for preliminary screening, and 3-4 positive monoclones were selected and amplified into 24-well plates to continue culture. After 2-3 days, FACS was used to confirm antigen binding positive. According to the detection results of 24-well plate samples, an optimal clone was selected for expansion culture, liquid nitrogen cryopreservation, antibody production and purification.

### (5) Production and purification of chimeric antibodies

The positive hybridoma cells selected above were cultured in T-75 culture flask with serum-free medium and passaged for 3 generations. When the hybridoma cells were in good condition, the cells were transferred to a 250 ml culture flask. 250 ml of DMEM + 2.5% FBS (low IgG) was adde to each flask to adjust the cell density to 1×10⁵ cells/mL. The culture flask was placed in a rotating incubator at 37 °C with the rotate speed at 3 rpm. The hybridoma cells were cultured for 14 days, the cell culture supernatant was collected, the cell components were removed by centrifugation, and the supernatant was filtered through a filter with a pore size at 0.45 µm.

Chimeric anti-GITR antibodies were purified from cell culture supernatants using a Protein G chromatography column (perchased from GE Healthcare). First, the column was equilibrated with an equilibrium solution (PBS buffer, pH 7.2), then the supernatant of the hybridoma culture was loaded, four column volumes were equilibrated, and eluted with an elution buffer (0.1 mol/L acetate buffer, pH 2.5). 10% neutralization buffer (1.0 mol/L Tris-HCl) was then added to the elution buffer for neutralization, and the sample was filtered through a filter with the pore size at 0.22 µm to obtain purified chimeric anti-GITR antibody with sterile filtration.

### EXAMPLE 2 Identification of chimeric anti-GITR antibody

### (1) Binding activity based on cell level

The pIRES plasmid containing the full-length nucleotide sequence encoding human GITR described in the step (2) in Example 1 was transfected into a 293F cell line to obtain a stable 293F cell line containing human GITR (herein referred to as HEK293-hGITR-4 stable cell line). The pIRES plasmid with the monkey-derived GITR full-length gene was transfected into the HEK293 cell line to construct a HEK293 stable cell line containing monkey GITR (herein referred to as HEK293-cGITR stable cell line), wherein the database accession number of the monkey GITR nucleotide sequence is XP_005545180.1. The tool antibody Tab9H6v3 was constructed with reference to the patent (US_2015_0064204 A1). The 293F-hGITR stable cell line and 293F-cGITR stable cell line were expanded in a T-75 cell culture flask to a confluence of 90%. The medium was aspirated, and the cells were incubated with chimeric anti-GITR antibody or IgG1 isotype control antibody in PBS+2% FBS for 1 hour so that the antibody binds to the GITR protein expressed on the cell surface. Cells were collected and washed, and then incubated with anti-human antibody conjugated with fluorescent dye at 4 °C for 1 hour. Mean fluorescence intensity (MFI) of chimeric anti-GITR antibodies bound to cells expressing hGITR or cGITR was determined by flow cytometry. The EC50 of the fitting curve was calculated by GraphPad Prism software. The results are shown in Table 2. The antibody to be tested can bind to human or monkey GITR protein on the cell surface. The data in the table is the average fluorescence intensity values of the cell populations measured by MFI.

**Table 2 FACS detection of binding reaction of chimeric anti-GITR antibody to 293F-hGITR or 293F-cGITR**

| Antibody ID | Clone ID | Binding to 293F-hGITR (EC50/nM) | Binding to 293F-cGITR (EC50/nM) |
|---|---|---|---|
| 3503-mAb019 | 96A10H9 | 0.070 | 0.010 |
| 3503-mAb064 | 265G9A11 | 0.385 | 0.404 |
| 3503-mAb070 | 272G5E1 | 0.358 | 0.198 |
| 3503-mAb076 | 277C12G4 | 0.152 | 0.226 |
| Tab9H6v3 | N/A | 0.190 | 0.160 |

The results of the above binding experiments show that the chimeric anti-GITR antibody has high binding activity to 293F-hGITR cells expressing human GITR, and has good cross-reactivity to 293F-cGITR cells expressing monkey GITR, which is reflected in the high binding activity to 293F-cGITR.

### (2) Activating NF-κB activity

Cells co-expressing NF-κB and hGITR (Jurkat-NF-κB-Luc-hGITR-3C8) were inoculated in 96-well cell culture plates at 2×10⁶ cells/ml, 50 µl/well. Then the cells were incubated with 50 µl crosslinking or non-crosslinking anti-GITR antibody at 37 °C, 5% CO₂ for 5 hours. Finally, 100 µl Luciferase detection buffer was added to each well, and after 5 minutes of shock lysis in the dark, the fluorescence intensity was detected using a Varioskan ™ Lux plate reader. The EC50 of the fitting curve was calculated by GraphPad Prism software. Tool antibodies Tab9H6v3 and Tab6C8 (US_8388967_B2) were used as positive controls. Results are shown in Table 3. The activation of NF-κB activity by the antibody to be tested was dependent on F (ab)₂.

**Table 3 Activation of NF-κB activity by chimeric anti-GITR antibody**

| | | | |
|---|---|---|---|
| Antibody ID | Clone ID | EC50 (nM) | |
| | | Non-coupling (Non-Crosslinking) | Coupling (Crosslinking) |
| 3503-mAb019 | 96A10H9 | N/A | 12.49 |
| 3503-mAb064 | 265G9A11 | >0.2 | 0.101 |
| 3503-mAb070 | 272G5E1 | 0.062 | >0.09 |
| 3503-mAb076 | 277C12G4 | >0.09 | >0.27 |
| Tab9H6v3 | N/A | 0.302 | 36.23 |

### (3) Activating T cell activity

Anti-human GITR antibody or IgG1 isotype control antibody was co-incubated with a mixture of 0.3 µg/ml OKT3 and 2.7 µg/ml anti-hFc antibody bound to plate at 37 °C for 30 minutes. The EasySep™ Human T cell isolation kit (Stemcell, Cat # 17951) was used to isolate and harvest T cell from human peripheral blood. 200 ul, 3×10⁵ T cells were added to each well at 37 °C and were incubated for 3 days. On the fifth day the cell culture supernatant was collected, and the INF-γ content in the culture supernatant was detected by Human IFN-γ ELISA Kit. The EC50 of the fitting curve was calculated by GraphPad Prism software. Results are shown in Table 4, the antibody to be tested activates T cell activity.

**Table 4 Chimeric anti-GITR antibodies activate T cell activity**

| Antibody ID | Clone ID | T cell activation (EC50/pM) |
|---|---|---|
| 3503-mAb019 | 96A10H9 | 7.8 |
| 3503-mAb064 | 265G9A11 | 0.3 |
| 3503-mAb070 | 272G5E1 | 3.1 |
| 3503-mAb076 | 277C12G4 | >2.5 |
| Tab9H6v3 | N/A | 4.0 |

These results indicate that chimeric anti-GITR antibody has the activity of activating T cells to synthesize and release INF-γ.

### Example 3 Determination of amino acid sequences of light and heavy chain variable regions

### (1) Isolation of total RNA

The lead antibody hybridoma cells obtained by screening were cultured and 5 × 10⁷ hybridoma cells were collected by centrifugation. The RNA was then extracted with reference to TRIzol ® Reagent's protocol. 1 ml Trizol was added to the cell precipitate, mixed and transferred to a 1.5 ml centrifuge tube and incubated at room temperature for 5 minutes. 0.2 ml of chloroform was then added to the sample and vortexed for 15 seconds. After standing for 2 minutes, the mixture was centrifuged at 12,000 g at 4 °C for 5 minutes. The supernatant was collected and transferred to a new 1.5 ml centrifuge tube, 0.5 ml isopropanol was added and gently mixed, and the sample was incubated at room temperature for 10 minutes. The sample was centrifuged at 12,000 g at 4 °C for 15 minutes. The supernatant was aspirated and the precipitate was washed with 1 ml of 75% (v/v) ethanol. The mixture was centrifuged at 12,000 g at 4 °C for 5 minutes, the supernatant was carefully discarded, and the precipitate was air-dried. Total RNA was obtained by dissolving the precipitate with water after DEPC treatment (water bath at 55 °C for 10 minutes).

### (2) Reverse transcription and PCR

Reverse transcription of RNA was performed according to the SMARTer ® RACE 5 '/3' Kit (Clontech, Cat # 634858) manual. Single strands of cDNA were synthesized using random decamer primers. The PCR program was set as follows: denaturation at 95 °C for 3 minutes, 35 denaturation cycles (95 °C for 30 seconds), annealing (55 °C for 30 seconds) and further extension (72 °C for 35 seconds), and then extension at 72 °C for 5 minutes. The PCR products was taken for agarose gel electrophoresis detection, and then the PCR products in the gel were purified by PureLink rapid gel extraction kit according to the kit instructions. Note: The extension temperature can be adjusted according to the actual situation.

### (3) Cloning and sequencing

The amplified DNA fragment clone was inserted into pMD ® 18-T vector, and ligation reaction was carried out in a 10 µl reaction system containing sample 50 ng , vector 50 ng, ligase 0.5 µl, and buffer 1 µl, and reacted at 16 °C for half an hour; 5µl of the ligation product was taken and added to 100µl of competent cells, ice bath for 5 minutes, then heat shock in a 42 °C water bath for 1 minute, and put back on ice for 1 minute, and added with 650µl antibiotic-free SOC medium. The cells were resuscitated on a shaker at 37 °C at 200 RPM for 30 min, taken out with 200µl and spreaded on LB solid medium containing antibiotics and incubated overnight at 37 °C in an incubator. On the next day, primers M13F and M13R on the T vector were used to prepare a 30µl PCR system. Colony PCR was performed, a pipette tip was used to dip the colony into the PCR reaction system and pipette, and 0.5µl was aspirated onto another LB solid petri dish containing antibiotics to preserve the strain. After the PCR reaction was over, 5µl of the reaction solution was take out for agar glycogel electrophoresis detection, and the positive samples were sequenced. Wherein, the steps of sequencing can be found in Kabat, Sequences of Proteins of Immunological Interest, National Institutes of Health, Bethesda, Md. (1991). The sequencing results are shown in Table 5A and Table 5B.

**Table 5A GITR antibody amino acid sequence numbers**

| Antibody ID | Clone ID | Heavy chain (CDR) | Light chain (CDR) |
|---|---|---|---|
| 3503-mAb019 | 96A10H9 | 1 | 5 |
| | | (CDR1: aa 31-35) | (CDR1: aa 24-34) |
| | | (CDR2: aa 50-66) | (CDR2: aa 50-56) |
| | | (CDR3: aa 99-113) | (CDR3: aa 89-97) |
| 3503-mAb070 | 272G5E1 | 9 | 13 |
| | | (CDR1: aa 31-35) | (CDR1: aa 24-34) |
| | | (CDR2: aa 50-66) | (CDR2: aa 50-56) |
| | | (CDR3: aa 99-111) | (CDR3: aa 89-97) |
| 3503-mAb076 | 277C12G4 | 17 | 21 |
| | | (CDR1: aa 31-35) | (CDR1: aa 24-34) |
| | | (CDR2: aa 50-66) | (CDR2: aa 50-56) |
| | | (CDR3: aa 95-111) | (CDR3: aa 89-97) |
| 3503-mAb064 | 265G9A11 | 25 | 29 |
| | | (CDR1: aa 31-35) | (CDR1: aa 24-34) |
| | | (CDR2: aa 50-66) | (CDR2: aa 50-56) |
| | | (CDR3: aa 99-111) | (CDR3: aa 89-97) |

**Table 5B GITR antibody gene sequence numbers**

| Antibody ID | Clone ID | Heavy chain (CDR) | Light chain (CDR) |
|---|---|---|---|
| 3503-mAb019 | 96A10H9 | 33 | 34 |
| | | (CDR1: nt 91-105) | (CDR1: nt 70-102) |
| | | (CDR2: nt 148-198) | (CDR2: nt 148-168) |
| | | (CDR3: nt 295-339) | (CDR3: nt 265-291) |
| 3503-mAb070 | 272G5E1 | 35 | 36 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |
| 3503-mAb076 | 277C12G4 | 37 | 38 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |
| 3503-mAb064 | 265G9A11 | 39 | 40 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |

Wherein, the numbers in Table 5 are the "SEQ ID No." in the sequence listing. For example, the nucleotide sequence encoding the heavy chain variable region (VH) of 96A10H9 is SEQ ID No.33 in the sequence listing.

Wherein the nucleotide sequence encoding VH-CDR1 of 96A10H9 is from position 91 to position 105 in SEQ ID No. 33; the nucleotide sequence encoding VH-CDR2 of 96A10H9 is from position 148 to position 198 in SEQ ID No. 33; the nucleotide sequence encoding VH-CDR3 of 96A10H9 is from position 295 to position 339 in SEQ ID No. 33; the nucleotide sequence encoding VL-CDR1 of 96A10H9 is from position 70 to position 102 in SEQ ID No. 34; the nucleotide sequence encoding VL-CDR2 of 96A10H9 is from position 148 to position 168 in SEQ ID No. 34; the nucleotide sequence encoding VL-CDR3 of 96A10H9 is from position 265 to position 291 in SEQ ID No. 34;
the nucleotide sequence encoding VH-CDR1 of 272G5E1 is from position 91 to position 105 in SEQ ID No. 35 in the sequence listing; the nucleotide sequence encoding VH-CDR2 of 272G5E1 is from position 148 to position 198 in SEQ ID No. 35 in the sequence listing; the nucleotide sequence encoding VH-CDR3 of 272G5E1 is from position 295 to position 333 in SEQ ID No. 35 in the sequence listing; the nucleotide sequence encoding VL-CDR1 of 272G5E1 is from position 70 to position 102 in SEQ ID No. 36 in the sequence listing; the nucleotide sequence encoding VL-CDR2 of 272G5E1 is from position 148 to position 168 in SEQ ID No. 36 in the sequence listing; the nucleotide sequence encoding VL-CDR3 of 272G5E1 is from position 265 to position 291 in SEQ ID No. 36 in the sequence listing;

The nucleotide sequence encoding VH-CDR1 of 277C12G4 is from position 91 to position 105 in SEQ ID No. 37 in the sequence list; the nucleotide sequence encoding VH-CDR2 of 277C12G4 is from position 148 to position 198 in SEQ ID No. 37 in the sequence list; the nucleotide sequence encoding VH-CDR3 of 277C12G4 is from position 295 to position 333 in SEQ ID No. 37 in the sequence list; the nucleotide sequence encoding VL-CDR1 of 277C12G4 is from position 70 to position 102 in SEQ ID No. 38 ; the nucleotide sequence encoding VL-CDR2 of 277C12G4 is from position 148 to 168 in SEQ ID No. 38 in the sequence list; the nucleotide sequence encoding VL-CDR3 of 277C12G4 is from position 265 to position 291 in SEQ ID No. 38 in the sequence list;

The nucleotide sequence encoding VH-CDR1 of 265G9A11 is from position 91 to position 105 in SEQ ID No. 39 in the sequence list; the nucleotide sequence encoding VH-CDR2 of 265G9A11 is from position 148 to position 198 in SEQ ID No. 39 in the sequence list; the nucleotide sequence encoding VH-CDR3 of 265G9A11 is from position 295 to position 333 in SEQ ID No. 39 in the sequence list; the nucleotide sequence encoding VL-CDR1 of 265G9A11 is from position 70 to position 102 in SEQ ID No. 40 ; the nucleotide sequence encoding VL-CDR2 of 265G9A11 is from position 148 to 168 in SEQ ID No. 40; the nucleotide sequence encoding VL-CDR3 of 265G9A11 is from position 265 to position 291 in SEQ ID No. 40.

### Example 4 Transformation and preparation of fully human antibody IgG

### (1) Plasmid construction and preparation

Purified anti-GITR antibody from the culture supernatant of hybridoma cells had been obtained in Example 2, and according to the sequencing results of Example 3, the sequences of heavy chain variable region and light chain variable region of anti-GITR antibody was clear. The heavy chain variable region sequence of the anti-GITR antibody was recombined into an expression vector containing the signal peptide and the human heavy chain antibody IgG1 constant region (the expression vector was purchased from Invitrogen), and the light chain variable region sequence of the anti-GITR antibody was recombined into an expression vector containing the signal peptide and the human antibody light chain kappa constant region. The recombinant plasmid was obtained and verified by sequencing (the sequencing method was the same as that in Example 3). Plasmids with a mass of more than 500µg were extracted using alkaline lysis kit (purchased from MACHEREY-NAGEL) to increase the purity, filtered through a 0.22 µm pore diameter filter membrane (purchased from Millopore) for transfection.

### (2) Cell transfection

FreeStyle ™ 293 cells were cultured in FreeStyle 293 medium at 37°C, 130 rpm, 8% CO₂. The cell density was adjusted to 1.0-1.2 × 10⁶ cells/ml. At first, the DNA plasmid was diluted to 100 ug/100 ml using a medium and was incubated for 5 minutes by gentle vortex. Then, PEI at a final concentration of 200 ug/100ml was added to the DNA plasmid, incubated for 5 minutes by gentle vortex and the mixture was incubated for 15 minutes at room temperature. At last, that DNA-PEI mixture was gently added dropwise to the cells. The next day, peptone was added to a final concentration of 0.5% (w/v). The cells were cultured continuously under the conditions of 37°C, 130 rpm, 8% CO₂. Subsequently, culture supernatants and cells were collected every day, and antibody/protein expression was monitored. At about 6-7 days, that cell culture was centrifuged (3,000 rpm, 30 min), the supernatant was collect and filtered through a 0.22 µm pore diameter filter to obtain a filtered cell supernatant for antibody purification.

### (3) Antibody purification

For continuously used endotoxin-free chromatography columns and Protein A stuffing (purchased from GE Healthcare), 0.1M NaOH was used for washing for 30 minutes, or 5 column volumes of 0.5M NaOH was used for washing. For long-term unused column materials and chromatography columns, at least 1M NaOH was used for soaking for 1 hour, and non-endotoxic water was used for rinsing to neutrality, and the column material was washed with 10 column volumes of 1% (v/v) TritonX-100. 5 column volumes of PBS (PBS phosphate buffer, pH 7.2) was used for equilibrate. The filtered cell supernatant obtained in step (2) was loaded on the column, and the flow-through liquid was collecetd if necessary. After the samples were loaded, the column was washed with 5 column volumes of PBS. Elution was performed with 5 column volumes of 0.1M pH3.0 Glycine-HCl, and the eluate was collected, and neutralized with 0.5 column volume of 1M Tris-HCl (1.5M NaCl) with pH 8.5, and fully human anti-GITR antibody was obtained. All the above-mentioned solutions required a fresh configuration. After the fully human anti-GITR antibodies harvested, they were dialyzed for 4 hours in 1×PBS to avoid endotoxin contamination. After dialysis, spectrophotometry or a kit was used to determine the concentration, andSDS-PAGE and SEC-HPLC were used to determine the purity of the antibody, and an endotoxin detection kit (purchased from Lonza) was used to detect the content of antibody endotoxin.

### EXAMPLE 5 Identification of fully human anti-GITR antibody

### (1) Binding activity based on protein level

According to Example 1, the sequence encoding amino acid sequence Gln20-Glu155 of the extracellular domain of the rhesus GITR protein (database accession number of the monkey GITR nucleotide acid sequence is XP_005545180.1) was cloned into a human IgG Fc fragment (hFc) pCpC vector (perchased from Invitrogen, V044-50), and the cGITR-ECD-hFc protein was obtained by transfection, purification, etc.

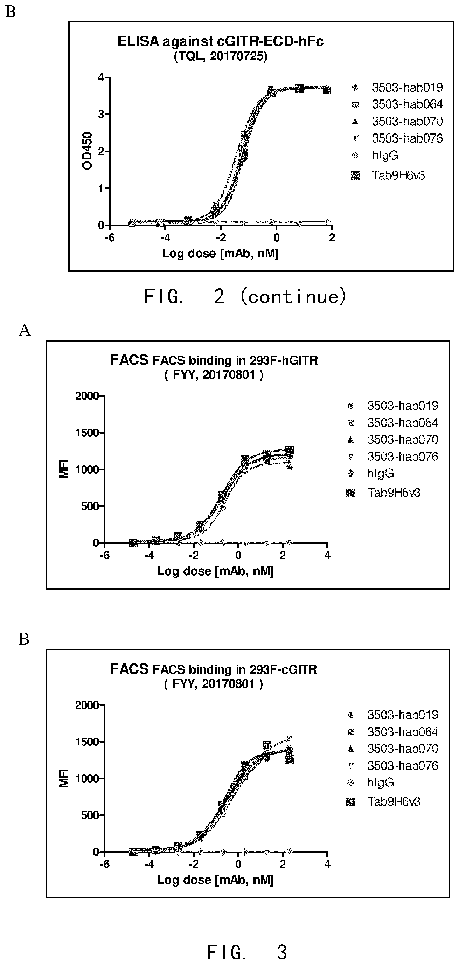

HGITR-ECD-hFc protein and cGITR-ECD-hFc protein were diluted to 1 µg/ml with PBS hydrochloric acid buffer (pH 7.2), and 100 µl dilution was added to 96-well ELISA microplate, and incubated at 4 °C overnight. The microplate was washed three times with 300 µl/well wash buffer PBST (PBS+0.05% Tween-20), 300 µl blocking buffer (PBST+1% BSA) was added to each well, and incubated at room temperature for 2 hours. The plate was washed three times, 100 µl, 10 µg/ml anti-GITR antibody was added to each well, and was incubated at 37 °C for 1 hour. The plate was washed three times, 100 µl horseradish peroxidase (HRP) labeled anti-human Fab secondary antibody was added to each well, and was incubated at 37 °C for half an hour. The plate was washed three times, 100 µl tetramethylbenzidine (TMB) was added to each well. After 15 minutes of color development, 50 µl IN HCl termination solution was added to each well, and the OD450 value was read with a plate reader. The EC50 of the binding curve was calculated by GraphPad Prism software. The results are shown in Table 6 and FIG. 2.

**Table 6 ELISA assay of binding activity of fully human anti-GITR antibody to hGITR-ECD-hFc and cGITR-ECD-hFc**

| Antibody ID | Clone ID | Binding to hGITR-ECD-hFc (EC50/nM) | Binding to cGITR-ECD-hFc (EC50/nM) |
|---|---|---|---|
| 3503-hab019 | 96A10H9 | 0.07913 | 0.07169 |
| 3503-hab064 | 265G9A11 | 0.04430 | 0.03694 |
| 3503-hab070 | 272G5E1 | 0.05436 | 0.05286 |
| 3503-hab076 | 277C12G4 | 0.05930 | 0.05512 |
| Tab9H6v3 | N/A | 0.07095 | 0.06369 |

The above results show that the fully human anti-GITR antibody in the present invention has strong binding activity to hGITR-ECD-hFc protein and good cross-binding activity to cGITR-ECD-hFc.

### (2) Binding activity based on cell level

The binding activity of the fully human anti-GITR antibody against 293F-hGITR stable cell lines and 293F-cGITR stable cell lines was detected by FACS according to the step (1) of Example 2, and the results were shown in Table 7 and FIG. 3.

**Table 7 FACS detection of the activity of the fully human anti-GITR antibody binding to 293F-hGITR and 293F-cGITR.**

| Antibody ID | Clone ID | Binding to 293F-hGITR (EC50/nM) | Binding to 293F-cGITR (EC50/nM) |
|---|---|---|---|
| 3503-hab019 | 96A10H9 | 0.2501 | 0.5296 |
| 3503-hab064 | 265G9A11 | 0.1943 | 0.2544 |
| 3503-hab070 | 272G5E1 | 0.2027 | 0.3009 |
| 3503-hab076 | 277C12G4 | 0.1730 | 0.5803 |
| Tab9H6v3 | N/A | 0.1840 | 0.2347 |

The above results show that the fully human anti-GITR antibody in the present invention has a binding activity similar to the tool antibody to the stable cell line 293F-hGITR expressing human GITR, and also has a good cross binding activity to the stable cell line 293F-cGITR expressing monkey GITR.

### (3) Activating NF-κB activity

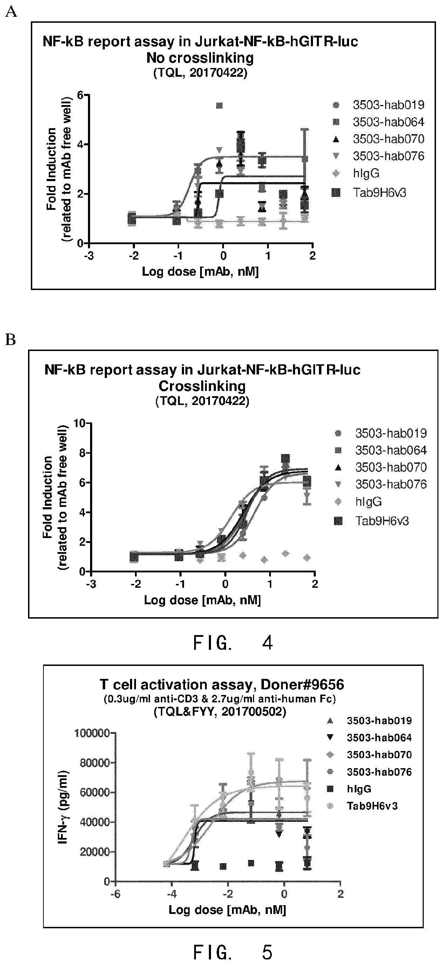

The activating activity of the fully human anti-GITR antibody against NF-κB was detected according to the step (2) of Example 2, and the results were shown in Table 8 and FIG. 4.

**Table 8 Fully human anti-GITR antibodies activate binding NF-κB activity**

| Antibody ID | Clone ID | EC50 (nM) | |
|---|---|---|---|
| | | Non-coupling | Coupling |
| 3503-hab019 | 96A10H9 | 0.2813 | 4.421 |
| 3503-hab064 | 265G9A11 | 0.1773 | 2.797 |
| 3503-hab070 | 272G5E1 | 0.2756 | 2.552 |
| 3503-hab076 | 277C12G4 | 0.1266 | 1.334 |
| Tab9H6v3 | N/A | 0.8076 | 3.086 |

The above results show that the fully human anti-GITR antibody in the present invention has the activity of activating NF-κB under both coupling and non-coupling conditions, and the activation effect under non-coupling conditions is better.

### (4) Activating T cell activity

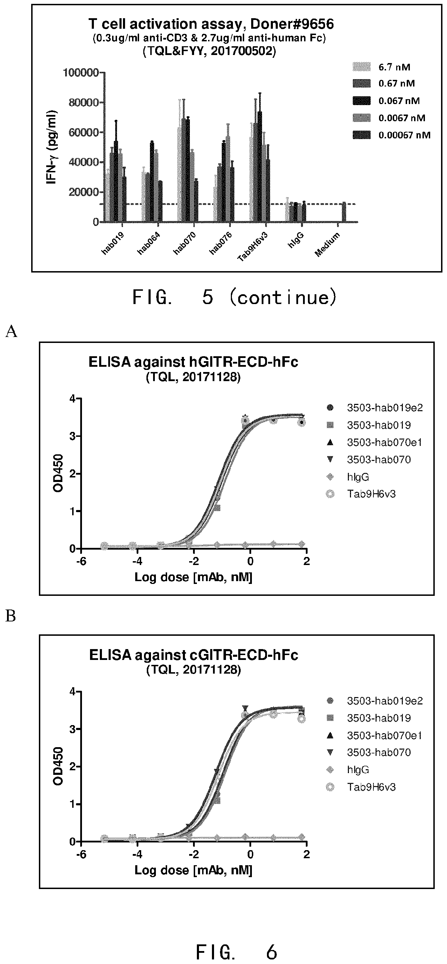

The activating activity of the fully human anti-GITR antibody against T cells was detected according to the step (3) of Example 2, and the results were shown in Table 9 and FIG. 5.

**Table 9 Fully human anti-GITR antibodies activate T cell activity**

| Antibody ID | Clone ID | T cell activation (EC50/pM) |
|---|---|---|
| 3503-hab019 | 96A10H9 | 0.6219 |
| 3503-hab064 | 265G9A11 | 0.6652 |
| 3503-hab070 | 272G5E1 | 2.5240 |
| 3503-hab076 | 277C12G4 | 0.5158 |
| Tab9H6v3 | N/A | 0.2259 |

The above results show that the fully human anti-GITR antibody in the present invention has the activity of activating T cells to synthesize and release TNF-γ.

### (5) Determination of binding dissociation equilibrium constants based on antigen

The antigen hGITR-ECD-hFc was immobilized on the chip surface in manual mode using the running buffer HBS-EP + (10mM HEPES, 150mM NaCl, 3mM EDTA, 0.05% P20, pH 7.4). First, a fresh mixture of 50 mM NHS and 200 mM EDC was used to activate the flow cell 4 of the tandem S CM5 sensor chip. The antigen was then diluted to 1 µg/ml with 10 mM NaAC (pH 5.0) and was injected into flow cell 4 for approximately 40 seconds. Finally, 1 M ethanolamine was used to block the remaining active coupling sites.

The antibody was diluted to a series of concentrations (0, 3.125, 6.25, 12.5, 25, 50 nM) using the running buffer HBS-EP + and injected into flow cells 1 and 4 at a flow rate of 30 µ L/min for 180 seconds. Buffer flow was maintained for 600 seconds to determine dissociation. To remove the test antibody from the surface, 10 mM Glycine-HCl (pH 1.5) was injected into flow cells 1 and 4 at a flow rate of 10 µ L/min for 30 seconds. The above steps were repeated for each concentration of continuously diluted antibodies.

The KD value of each antibody was evaluated using Biacore T200 evaluation software 3.0, where flow cell 1 was used as a reference flow cell. The results are shown in Table 10.

**Table 10 Binding kinetics and affinity of fully human anti-GITR antibody to hGITR-ECD-hFc protein**

| Antibody ID | Clone ID | Antigen | ka (1/ms) | kd (1/s) | KD (M) |
|---|---|---|---|---|---|
| 3503-hab019 | 96A10H9 | hGITR-ECD-hFc | 5.115E+05 | 2.051E-04 | 4.009E-10 |
| 3503-hab064 | 265G9A11 | | 5.323E+05 | 3.153E-04 | 5.924E-10 |
| 3503-hab070 | 272G5E1 | | 7.373E+05 | 1.849E-04 | 2.507E-10 |
| 3503-hab076 | 277C12G4 | | 6.636E+05 | 6.842E-04 | 1.031E-09 |
| Tab9H6v3 | N/A | | 6.197E+05 | 8.838E-05 | 1.426E-10 |

The above results show that the KD values of the fully human anti-GITR antibody and the hGITR-ECD-hFc protein in the present invention are all at the nanomole level, indicating that these antibodies have good affinity for hGITR-ECD-hFc.

### EXAMPLE 6 Design and preparation of engineered fully human anti-GITR antibody

### (1) Engineering modification of fully human anti-GITR antibody design

The CDR region sequences of the above fully human anti-GITR antibodies were analyzed, and the hot spots in the sequence were removed by engineering modification technology. At the same time, germline sequence alignment analysis of the antibodies were performed to restore the mutation in the Frame work region. The engineered fully human anti-GITR antibody gene sequences are shown in Table 11, and the amino acid sequences are shown in Table 12.

**Table 11 Humanized anti-GITR antibody gene sequence numbers**

| Antibody ID | Clone ID | Heavy chain (CDR) | Light chain (CDR) |
|---|---|---|---|
| 3503-hab0 19e1 | 96A10H9 | 50 | 34 |
| | | (CDR1: nt 91-105) | (CDR1: nt 70-102) |
| | | (CDR2: nt 148-198) | (CDR2: nt 148-168) |
| | | (CDR3: nt 295-339) | (CDR3: nt 265-291) |
| 3503-hab0 19e2 | | 51 | 34 |
| | | (CDR1: nt 91-105) | (CDR1: nt 70-102) |
| | | (CDR2: nt 148-198) | (CDR2: nt 148-168) |
| | | (CDR3: nt 295-339) | (CDR3: nt 265-291) |
| 3503-hab0 19e3 | | 52 | 34 |
| | | (CDR1: nt 91-105) | (CDR1: nt 70-102) |
| | | (CDR2: nt 148-198) | (CDR2: nt 148-168) |
| | | (CDR3: nt 295-339) | (CDR3: nt 265-291) |
| 3503-hab070e1 | 272G5E1 | 35 | 55 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |
| 3503-hab076e1 | 277C12G4 | 37 | 56 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |
| 3503-hab064e1 | 265G9A11 | 53 | 54 |
| | | (CDR1: nt 91-105) | (CDR1: nt 70-102) |
| | | (CDR2: nt 148-198) | (CDR2: nt 148-168) |
| | | (CDR3: nt 295-333) | (CDR3: nt 265-291) |

Wherein, the numbers in Table 11 are the "SEQ ID No." in the sequence listing. For example, the nucleotide sequence encoding the heavy chain variable region (VH) of the engineered antibody 3503-hab019e1 is SEQ ID No.50 in the sequence listing.

Wherein, the nucleotide sequence encoding VH-CDR1 of 3503-hab019e1 is from position 91 to position 105 in SEQ ID No. 50 in the sequence list; the nucleotide sequence encoding VH-CDR2 of 3503-hab019e1 is from position 148 to position 198 in SEQ ID No. 50 in the sequence list; the nucleotide sequence encoding VH-CDR3 of 3503-hab019e1 is from position 295 to position 339 in SEQ ID No. 50 in the sequence list; the nucleotide sequence encoding VL-CDR1 of 3503-hab019e1 is from position 70 to position 102 in SEQ ID No. 34; the nucleotide sequence encoding VL-CDR2 of 3503-hab019e1 is from position 148 to 168 in SEQ ID No. 34 in the sequence list; the nucleotide sequence encoding VL-CDR3 of 3503-hab019e1 is from position 265 to position 291 in SEQ ID No. 34 in the sequence list;

The nucleotide sequence encoding VH-CDR1 of 3503-hab019e2 is from position 91 to position 105 in SEQ ID No. 51 in the sequence list; the nucleotide sequence encoding VH-CDR2 of 3503-hab019e2 is from position 148 to position 198 in SEQ ID No. 51 in the sequence list; the nucleotide sequence encoding VH-CDR3 of 3503-hab019e2 is from position 295 to position 339 in SEQ ID No. 51 in the sequence list; the nucleotide sequence encoding VL-CDR1 of 3503-hab019e2 is from position 70 to position 102 in SEQ ID No. 34 ; the nucleotide sequence encoding VL-CDR2 of 3503-hab019e2 is from position 148 to 168 in SEQ ID No. 34 in the sequence list; the nucleotide sequence encoding VL-CDR3 of 3503-hab019e2 is from position 265 to position 291 in SEQ ID No. 34 in the sequence list;

The nucleotide sequence encoding VH-CDR1 of 3503-hab019e3 is from position 91 to position 105 in SEQ ID No. 52 in the sequence list; the nucleotide sequence encoding VH-CDR2 of3503-hab019e3 is from position 148 to position 198 in SEQ ID No. 52 in the sequence list; the nucleotide sequence encoding VH-CDR3 of3503-hab019e3 is from position 295 to position 339 in SEQ ID No. 52 in the sequence list; the nucleotide sequence encoding VL-CDR1 of 3503-hab019e3 is from position 70 to position 102 in SEQ ID No. 34 ; the nucleotide sequence encoding VL-CDR2 of 277C12G4 is from position 148 to 168 in SEQ ID No. 34 in the sequence list; the nucleotide sequence encoding VL-CDR3 of3503-hab019e3 is from position 265 to position 291 in SEQ ID No. 34 in the sequence list;

The nucleotide sequence encoding VH-CDR1 of 3503-hab070e1 is from position 91 to position 105 in SEQ ID No. 35 in the sequence list; the nucleotide sequence encoding VH-CDR2 of 3503-hab070e1 is from position 148 to position 198 in SEQ ID No. 35 in the sequence list; the nucleotide sequence encoding VH-CDR3 of3503-hab070e1 is from position 295 to position 333 in SEQ ID No. 35 in the sequence list; the nucleotide sequence encoding VL-CDR1 of 3503-hab070e1 is from position 70 to position 102 in SEQ ID No. 55; the nucleotide sequence encoding VL-CDR2 of 3503-hab019e1 is from position 148 to 168 in SEQ ID No. 55 in the sequence list; the nucleotide sequence encoding VL-CDR3 of 3503-hab019e1 is from position 265 to position 291 in SEQ ID No. 55 in the sequence list;

The nucleotide sequence encoding VH-CDR1 of 3503-hab076e1 is from position 91 to position 105 in SEQ ID No. 37 in the sequence list; the nucleotide sequence encoding VH-CDR2 of 3503-hab076e1 is from position 148 to position 198 in SEQ ID No. 37 in the sequence list; the nucleotide sequence encoding VH-CDR3 of 3503-hab076e1 is from position 295 to position 333 in SEQ ID No. 37 in the sequence list; the nucleotide sequence encoding VL-CDR1 of 3503-hab076e1 is from position 70 to position 102 in SEQ ID No. 56 ; the nucleotide sequence encoding VL-CDR2 of 3503-hab076e1 is from position 148 to 168 in SEQ ID No. 56 in the sequence list; the nucleotide sequence encoding VL-CDR3 of 3503-hab076e1 is from position 265 to position 291 in SEQ ID No. 56 in the sequence list;

The nucleotide sequence encoding VH-CDR1 of 3503-hab064e1 is from position 91 to position 105 in SEQ ID No. 53 in the sequence list; the nucleotide sequence encoding VH-CDR2 of 3503-hab064e1 is from position 148 to position 198 in SEQ ID No. 53 in the sequence list; the nucleotide sequence encoding VH-CDR3 of 3503-hab064e1 is from position 295 to position 333 in SEQ ID No. 53 in the sequence list; the nucleotide sequence encoding VL-CDR1 of 3503-hab064e1 is from position 70 to position 102 in SEQ ID No. 54 ; the nucleotide sequence encoding VL-CDR2 of 3503-hab019e2 is from position 148 to 168 in SEQ ID No. 54 in the sequence list; the nucleotide sequence encoding VL-CDR3 of 3503-hab064e1 is from position 265 to position 291 in SEQ ID No. 54 in the sequence list;

**Table 12 Engineered fully human anti-GITR antibody amino acid sequence numbers**

| Antibody ID | Clone ID | Heavy chain (CDR) | Light chain (CDR) |
|---|---|---|---|
| 3503-hab019e1 | 96A10H9 | 41 | 5 |
| | | (2: CDR1) | (6: CDR1) |
| | | (3: CDR2) | (7: CDR2) |
| | | (4: CDR3) | (8: CDR3) |
| 3503-hab019e2 | | 42 | 5 |
| | | (2: CDR1) | (6: CDR1) |
| | | (3: CDR2) (43: CDR3) | (7: CDR2) (8: CDR3) |
| 3503-hab019e3 | | 44 | 5 |
| | | (2: CDR1) | (6: CDR1) |
| | | (3: CDR2) | (7: CDR2) |
| | | (45: CDR3) | (8: CDR3) |
| 3503-hab070e1 | 272G5E1 | 9 | 48 |
| | | (10: CDR1) | (14: CDR1) |
| | | (11: CDR2) | (15: CDR2) |
| | | (12: CDR3) | (16: CDR3) |
| 3503-hab076e1 | 277C12G4 | 17 | 49 |
| | | (18: CDR1) | (22: CDR1) |
| | | (19: CDR2) | (23: CDR2) |
| | | (20: CDR3) | (24: CDR3) |
| 3503-hab064e1 | 265G9A11 | 46 | 47 |
| | | (26: CDR1) | (30: CDR1) |
| | | (27: CDR2) | (31: CDR2) |
| | | (28: CDR3) | (32: CDR3) |

Wherein, the numbers in Table 12 are the "SEQ ID No." in the sequence listing. For example, the nucleotide sequence encoding the heavy chain variable region of the engineered antibody 3503-hab019e1 is SEQ ID No.41 in the sequence listing.

Wherein, the amino acid sequences of VH-CDR1, VH-CDR2 and VH-CDR3 of antibody 3503-hab019e1 are SEQ ID No. 2, SEQ ID No. 3 and SEQ ID No. 4, respectively; the amino acid sequences of VL-CDR1, VL-CDR2 and VL-CDR3 of antibody 3503-hab019e1 are SEQ ID No. 6, SEQ ID No. 7 and SEQ ID No. 8, respectively;
the amino acid sequences of VH-CDR1, VH-CDR2 and VH-CDR3 of antibody 3503-hab019e2 are SEQ ID No. 2, SEQ ID No. 3 and SEQ ID No. 43, respectively; the amino acid sequences of VL-CDR1, VL-CDR2 and VL-CDR3 of antibody 3503-hab019e2 are SEQ ID No. 6, SEQ ID No. 7 and SEQ ID No. 8, respectively;
the amino acid sequences of VH-CDR1, VH-CDR2 and VH-CDR3 of antibody 3503-hab019e3 are SEQ ID No. 2, SEQ ID No. 3 and SEQ ID No. 45, respectively; the amino acid sequences of VL-CDR1, VL-CDR2 and VL-CDR3 of antibody 3503-hab019e3 are SEQ ID No. 6, SEQ ID No. 7 and SEQ ID No. 8, respectively;
the amino acid sequences of VH-CDR1, VH-CDR2 and VH-CDR3 of antibody 3503-hab070e1 are SEQ ID No. 10, SEQ ID No. 11 and SEQ ID No. 12, respectively; the amino acid sequences of VL-CDR1, VL-CDR2 and VL-CDR3 of antibody 3503-hab070e1 are SEQ ID No. 14, SEQ ID No. 15 and SEQ ID No. 16, respectively;
the amino acid sequences of VH-CDR1, VH-CDR2 and VH-CDR3 of antibody 3503-hab076e1 are SEQ ID No. 18, SEQ ID No. 19 and SEQ ID No. 20, respectively; the amino acid sequences of VL-CDR1, VL-CDR2 and VL-CDR3 of antibody 3503-hab076e1 are SEQ ID No. 22, SEQ ID No. 23 and SEQ ID No. 24, respectively;
the amino acid sequences of VH-CDR1, VH-CDR2 and VH-CDR3 of antibody 3503-hab064e1 are SEQ ID No. 26, SEQ ID No. 27 and SEQ ID No. 28, respectively; the amino acid sequences of VL-CDR1, VL-CDR2 and VL-CDR3 of antibody 3503-hab064e1 are SEQ ID No. 30, SEQ ID No. 31 and SEQ ID No. 32, respectively.

### (2) Preparation of engineered fully human anti-GITR antibody

The engineered fully human anti-GITR antibody was prepared with reference to Example 4.

### EXAMPLE 7 Identification of engineered fully human anti-GITR antibody

### (1) Binding activity based on protein level

The binding activity of the engineered fully human anti-GITR antibody to the hGITR-ECD-hFc protein or the cGITR-ECD-hFc protein was detected by an enzyme-linked immunosorbent assay iaccording to the step (1) of Example 5. The results are shown in Table 13 and FIG. 6.

**Table 13 ELISA determination of binding activity of engineered fully humanized anti-GITR antibody to hGITR-ECD-hFc and cGITR-ECD-hFc**

| Antibody ID | Clone ID | Binding to hGITR-ECD-hFc (EC50/nM) | Binding to cGITR-ECD-hFc (EC50/nM) |
|---|---|---|---|
| 3503-hab019e2 | 96A10H9 | 0.089 | 0.096 |
| 3503-hab019 | | 0.110 | 0.115 |
| 3503-hab070e1 | 272G5E1 | 0.074 | 0.062 |
| 3503-hab070 | | 0.078 | 0.063 |
| Tab9H6v3 | N/A | 0.083 | 0.073 |

The above results show that the engineered fully human anti-GITR antibody obtained through engineering modification in the present invention has similar binding activity to tool antibody against hGITR-ECD-hFc protein, and good cross-binding activity to cGITR-ECD-hFc protein.

### (2) Binding activity based on cell level

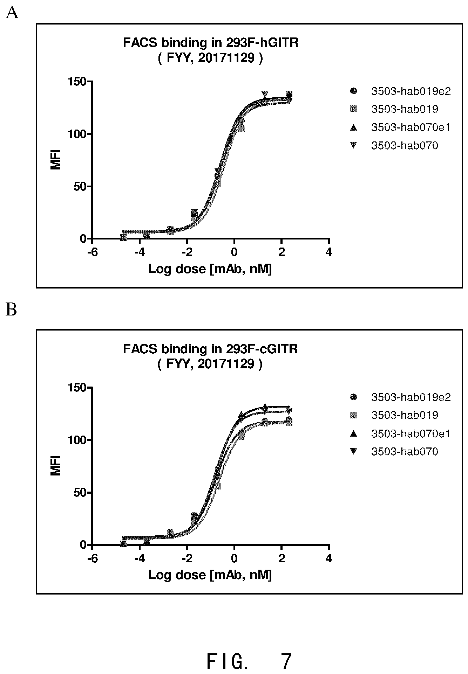

The binding activity of the engineered fully human anti-GITR antibody against 293F-HGITR stable cell lines and 293F-CGITR stable cell lines was detected by FACS according to the step (1) of Example 2, and the results are shown in Table 14 and FIG. 7.

**Table 14 FACS detection of the activity of the engineered fully human anti-GITR antibody binding to 293F-hGITR and 293F-cGITR.**

| Antibody ID | Clone ID | Binding to 293F-hGITR (EC50/nM) | Binding to 293F-cGITR (EC50/nM) |
|---|---|---|---|
| 3503-hab019e2 | 96A10H9 | 0.155 | 0.310 |
| 3503-hab019 | | 0.222 | 0.418 |
| 3503-hab070e1 | 272G5E1 | 0.169 | 0.264 |
| 3503-hab070 | | 0.141 | 0.258 |

The above results show that the binding activity of the fully human anti-GITR antibody obtained by engineering modification in the present invention to the stable cell line 293F-hGITR expressing the human GITR gene and the stable cell line 293F-cGITR expressing the monkey GITR gene is similar to or higher than the binding activity before modification.

### (3) Activating NF-κB activity

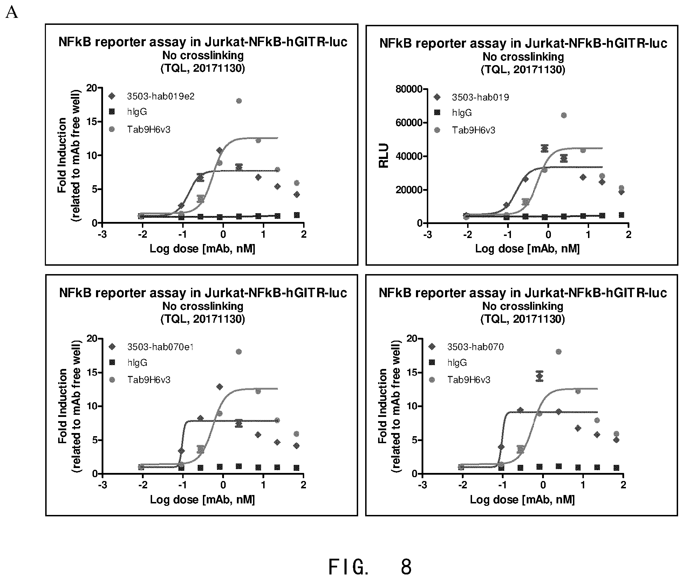

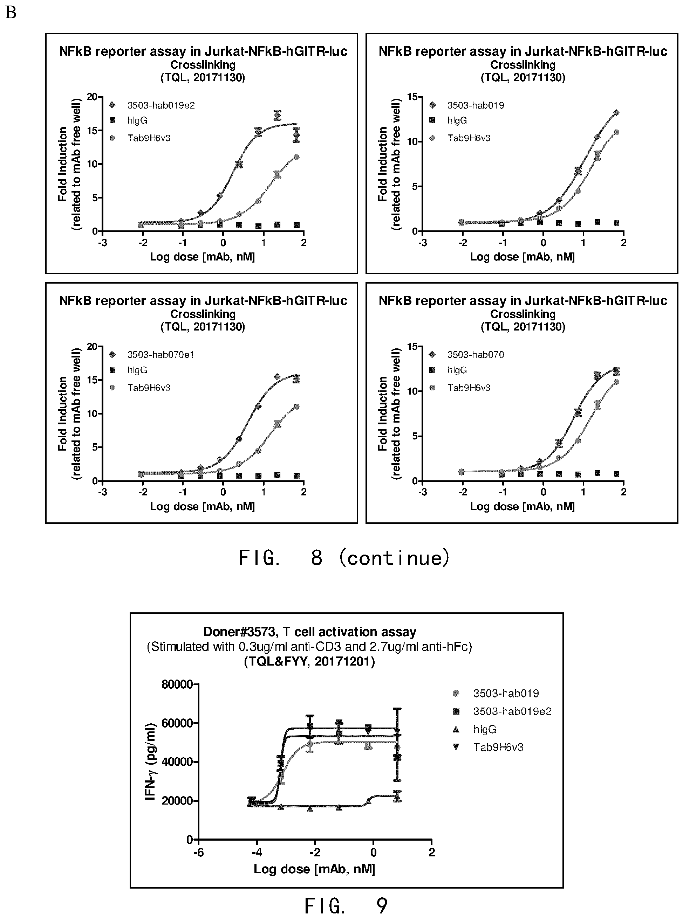

The activating activity of the engineered fully human anti-GITR antibody against NF-κB was detected according to the step (2) of Example 2, and the results were shown in Table 15 and FIG. 8.

**Table 15 Engineered fully human anti-GITR antibodies activate NF-κB activity**

| Antibody ID | Clone ID | EC50 (nM) | |
|---|---|---|---|
| | | Non-coupling | Coupling |
| 3503-hab019e2 | 96A10H9 | 0.1387 | 1.740 |
| 3503-hab019 | | 0.1659 | 10.87 |
| 3503-hab070e1 | 272G5E1 | 0.0957 | 4.079 |
| 3503-hab070 | | 0.0959 | 5.782 |
| Tab9H6v3 | N/A | 0.5521 | 14.970 |

The above results show that the fully human anti-GITR antibody obtained by engineering modification in the present invention has better activation activity to NF-κB under both coupling and non-coupling conditions. Wherein, the activation activity of the antibody obtained by engineering modification is better than that of the antibody before modification, and both are obviously better than that of the tool antibody Tab9H6v3.

### (4) Activating T cell activity

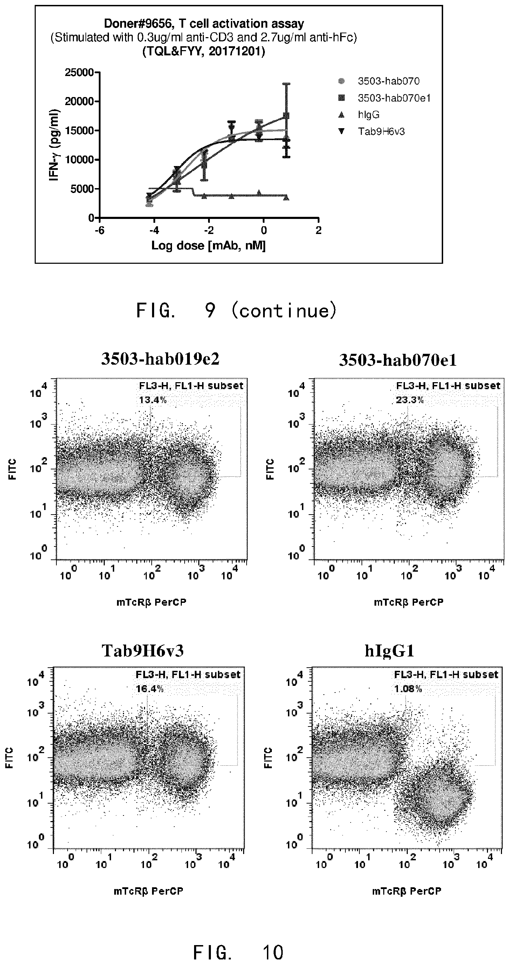

The activating activity of the engineered fully human anti-GITR antibody against T cells was detected according to the step (3) of Example 2, and the results were shown in Table 16 and FIG. 9.

**Table 16 Engineered fully humanized anti-GITR antibodies activate T cell activity**

| Antibody ID | Clone ID | T cell activation (EC50/pM) |
|---|---|---|
| 3503-hab019 | 96A10H9 | 0.8047 |
| 3503-hab019e2 | | 0.6348 |
| 3503-hab070 | 272G5E1 | 1.2220 |
| 3503-hab070e1 | | 3.7780 |

The above results show that the fully human anti-GITR antibody obtained by engineering modification in the present invention has the same activity as the antibody before modification in activating T cells to synthesize and release TNF-y.

### (5) Determination of binding dissociation equilibrium constants based on antigen

Binding kinetics and affinity of the engineered fully human anti-GITR antibody to the antigen hGITR-ECD-hFc protein were tested by Biacore according to step (5) of Example 5, and the results are shown in Table 17.

**Table 17 Binding kinetics and affinity of engineered fully human anti-GITR antibodies to hGITR-ECD-hFc protein**

| **Antibody ID** | **Clone ID** | **Antigen** | **ka (1/ms)** | **kd (1/s)** | **KD (M)** |
|---|---|---|---|---|---|
| 3503-hab019e2 | 96A10H9 | hGITR-ECD-hFc | 5.996E+5 | 2.302E-4 | 3.839E-10 |
| 3503-hab070e1 | 272G5E1 | | 7.104E+5 | 3.812E-4 | 5.367E-10 |
| Tab9H6v3 | N/A | | 5.203E+5 | 7.000E-5 | 1.345E-10 |

The above results indicate that the fully human anti-GITR antibodies 3503-hab019e2 and 3503-hab070e1 obtained by engineering modification in the present invention have similar binding dissociation equilibrium constants (KD (M)) to the tool antibody Tab9H6v3.

### EXAMPLE 8 Tumor growth inhibition activity of engineered fully human anti-GITR antibody

### (1) The binding activity of engineered fully human anti-GITR antibody to humanized mouse spleen cells

15 µg of anti-mCD3 antibody was dissolved in 400 µl PBS. Humanized B-hGITR mice were captured and injected 200 µl intraperitoneally. After 24h, the mice were euthanized by CO₂ and their spleens were quickly extracted and placed into a 15 mL centrifuge tube containing 5 mL PBS. Then the spleen was transferred to a 6-well plate and placed on a 70-meter screen, 1 ml PBS was added, the tail of a sterile syringe was used to grind, and then the screen was washed with 2 ml PBS; the filtered cell suspension was centrifuged at 3,000 rpm for 5 min, then the supernatant was discarded, and then the supernatant was left instantly; 1 ml red blood cell lysate was added to each spleen and lysed on ice for 5 min; 5 ml PBS was added and the cells were mixed well; the cells were centrifuged at 4 °C for 3,000 rpm for 3 min, then the supernatant was discarded, and then the supernatant was left instantly; the cells were resuspended with 300 µL PBS, blocked with 6 µL anti-Mcd16/32 and incubated on ice for 15 min; then the cells were mixed with 300 µl PBS. Fluorescence intensity was detected by FACS after flow staining. The FACS detection results are shown in FIG. 10.

The results show that 3503-hab019e2 and 3503-hab070e1 antibodies have similar binding activity to GITR humanized mouse spleen cells as Tab9H6v3, while the isotype control IgG has no binding activity to GITR humanized mouse spleen cells.

### (2) Evaluation of anti-tumor activity in vivo of engineered human anti-GITR antibody in mice

The MC38 syngeneic mouse model was used to evaluate the anti-tumor activity *in vivo* of antibodies in mice using C57BL/6 mice (B-hGITR mice, Beijing White Osetu Gene Biotechnology Co., Ltd.) knocked-in with human GITR gene. The experiment was designed as following: 24 human GITR knock-in C57BL/6 mice were selected and divided into 4 groups, each with 6 mice. Tab9H6v3 and the isotype antibody hIgG1 were used as control. The samples were 3503-hab019e2 and 3503-hab070e1. The route of administration was intraperitoneal injection, the dosage was 10 mg/kg, once every 3 days, a total of 6 doses. After the administration, the observation lasted for two weeks. The body weight of mice was weighed every week, and the tumor volume was measured twice with vernier caliper, and the long diameter and short diameter of the tumor were measured. The volume calculation formula was: tumor volume = 0.5 × long diameter × short diameter².

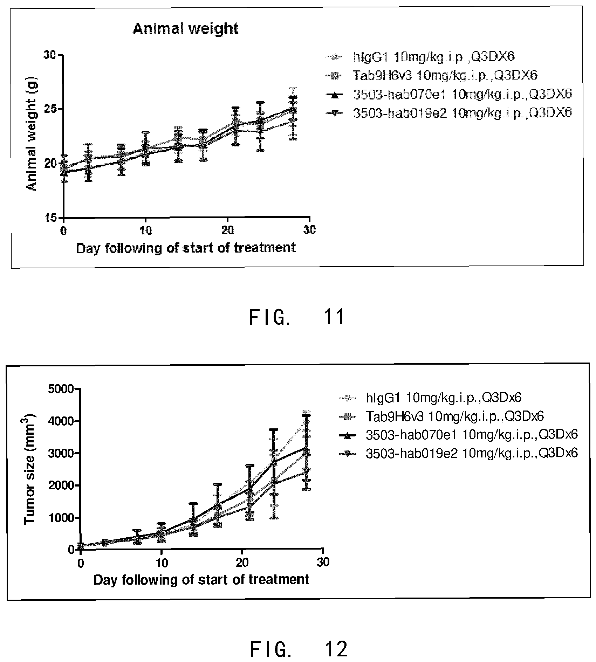

All the experimental animals were in good activity and feeding status during the administration, and their body weight increased to a certain extent. There was no significant difference in animal body weight between the test group and the control group 21 days after administration (P > 0.05). The body weight changes of all animals are shown in Table 18 and FIG. 11.

**Table 18 Effect of the test substance on body weight of B-hGITR mice transplanted with MC38 cells**

| | Body Weight (g)^{a} | | | 21 days after administration Body Weight change (g) |
|---|---|---|---|---|
| Groups | Before administration | Initial administration 21 days later | *P*^{b} | |
| hIgG1 | 19.4±0.4 | 23.3±0.3 | - | +3.9 |
| Tab9H6v3 | 19.6±0.3 | 23.8±0.4 | 0.266 | +4.2 |
| 3503-hab070e1 | 19.2±0.4 | 23.4±0.7 | 0.845 | +4.2 |
| 3503-hab019e2 | 19.6.±0.5 | 23.0±0.6 | 0.723 | +3.4 |

| | | | | |
|---|---|---|---|---|
| Note: a: mean ± standard error; b: the body weight of the administration group and the body weight of the solvent control group were statistically compared after 21 days of administration, t-test. | | | | |

During the experiment, all animals were closely monitored for tumor growth. The tumor was measured twice a week. The results were recorded and the tumor inhibition rate (TGI%) was calculated. Statistical analysis between groups was performed according to the tumor volume. The results are shown in Table 19 and Table 20.

**Table 19 Effect of anti-GITR antibody on tumor volume of MC38 cells transplanted B-hGITR mice**

| Groups | Tumor volume (mm³)^{a} | | | *P*^{b} |
|---|---|---|---|---|
| | Before administration | Initial administration 21 days later | TGI (%) | |
| hIgG1 | 126 ± 6 | 2056 ± 208 | - | - |
| Tab9H6v3 | 126 ± 6 | 1569 ± 217 | 25.3 | 0.136 |
| 3503-hab070e1 | 126 ± 5 | 1864 ± 294 | 10.0 | 0.605 |
| 3503-hab019e2 | 126 ± 5 | 1317 ± 167 | 38.3 | 0.019 |

| | | | | |
|---|---|---|---|---|
| Note: a: mean ± standard error; b: the tumor volume of the administration group and the tumor volume of the solvent control group were statistically compared after 21 days of administration, t-test. | | | | |

**Table 20 Statistical differences in tumor volume among groups^{a}**

| Groups | Tab9H6v3 | 3503-hab019e2 |
|---|---|---|
| 3503-hab070e1 | 0.438 | 0.136 |
| Tab9H6v3 | - | 0.378 |
| 3503-hab019e2 | - | - |

| | | |
|---|---|---|
| Note: a: the tumor volume of each groups were statistically compared after 21 days of administration, t-test. | | |

The results show that after 21 days of administration, the TGI% of the test sample 3503-hab070e1 group and Tab9H6v3 group were 10.0% and 25.3%, respectively. Compared with the hIgG1 control group, P > 0.05, indicating that GITR antibodies 3503-hab070e1 and Tab9H6v3 had no significant inhibitory effect on tumor growth. The TGI% of the test sample 3503-hab019e2 group was 38.3%. Compared with the hIgG1 control group, P<0.05, indicating that the GITR antibody 3503-hab019e2 has a significant inhibitory effect on tumor growth. In addition, a comparison of tumor volume among Tab9H6v3, 3503-hab070e1 and 3503-hab019e2 showed that there was no significant difference in tumor volume among the three groups after administration (P > 0.05). The results are shown in FIG. 12.

In this study, animals in each group were euthanized 29 days after the first administration, and tumors were stripped and weighed. The average value of tumor weight results for each group is shown in Table 21.

**Table 21 Inhibitory effect of anti-GITR antibody on tumor weight of MC38 colon cancer transplanted B-hGITR humanized mice**

| Groups | Number of animals (number) | Tumor weight (g) ^{A} | *P*^{b} |
|---|---|---|---|
| hIgG1 | 6 | 5.229 ± 0.652 | - |
| Tab9H6v3 | 6 | 4.261 ± 0.337 | 0.22 |
| 3503-hab070e1 | 6 | 5.909 ± 0.819 | 0.53 |
| 3503-hab019e2 | 6 | 3.824 ± 0.449 | 0.11 |

| | | | |
|---|---|---|---|
| Note: a: mean ± standard error. | | | |

After data analysis, the average tumor weight of Tab9H6v3 group and 3503-hab019e2 group decreased to a certain extent compared with the hIgG1 control group, but there was no significant difference (P > 0.05), indicating that anti-GITR antibody 3503-hab019e2 is similar to Tab9H6v3, and has a tendency to inhibit tumor growth.

### EXAMPLE 9 Study on that stability of engineered fully human anti-GITR antibody

### (1) Sample treatment and physical and chemical properties determination

20 mM histidine system containing 6% trehalose and 0.01% Tween 80, pH5.5 were selected as the buffer system for stability investigation. The sample was concentrated and dialyzed into the buffer system, and the protein content after dialysis was 20.3 mg/ml.

The isoelectric points of candidate antibodies were investigated by iCIEF. The isoelectric points of antibody molecules were between 9.0 and 9.5, and the isoelectric point of the main peak was 9.3.

The free sulfhydryl content of the sample detected by Gayman sulfhydryl detection kit was 0.83%.

The Tonset and Tm values of the candidate antibodies were analyzed by Micro-DSC, and the thermal stability was good. The results are shown in Table 22.

**Table 22 Tonset and Tm value of engineered fully humanized anti-GITR antibody**

| Antibody ID | Buffer system (Buffer) | Tₘ (°C) | | Tₒₙₛₑₜ (°C) |
|---|---|---|---|---|
| | | Tₘ₁ | Tₘ₂ | |
| 3503-hab019e2 | 20mM His, pH 5.5 | 65.18 | 77.44 | 58.57 |
| 3503-hab019e2 | 20mM His, pH 5.5, 6% Trehalose, 0.01% Tween-80 | 66.41 | 78.06 | 57.67 |

The above results show that the fully human anti-GITR antibody 3503-hab019e2 obtained by engineering modification in the present invention has good thermal stability in the above two different buffer systems.

### (2) Stability investigation under repeated freezing and thawing, different temperatures and different pH conditions

The effects of repeated freezing and thawing, different temperatures and different pH values on the stability of samples were investigated at the concentration of 20.3 mg/ml of engineered human anti-GITR antibody. The SEC and CEX test results of the treated samples are shown in Table 23 below.

**Table 23 Stability investigation results of low concentration samples**

| Sample Processing Information | SEC | | | CEX | | |
|---|---|---|---|---|---|---|
| | Polymer peak (%) | Monomer peak (%) | Degradation peak (%) | Acid peak (%) | Main peak (%) | Alkalin e peak (%) |
| Zero sample | 2.3 | 97.7 | 0.1 | 24.6 | 51.0 | 24.5 |
| Freeze and thaw 3 times | 2.3 | 97.6 | 0.1 | 23.9 | 51.0 | 25.2 |
| Freeze and thaw 6 times | 2.3 | 97.6 | 0.1 | 27.6 | 47.9 | 24.5 |
| 5 ± 3 °C for 4 weeks | 2.4 | 97.6 | 0.1 | 24.2 | 50.5 | 25.3 |
| 5 ± 3 °C for 6 weeks | 2.4 | 97.6 | 0.1 | 22.9 | 51.2 | 25.9 |
| 25 ± 2 °C/60% ± 5% RH for 2 weeks | 2.3 | 97.6 | 0.1 | 25.5 | 50.4 | 24.1 |
| 25 ± 2 °C/60% ± 5% RH for 4 weeks | 2.3 | 96.2 | 1.5 | 24.3 | 52.5 | 23.2 |
| 25 ± 2 °C/60% ± 5% RH for 6 weeks | 2.3 | 95.9 | 1.8 | 25.7 | 52.5 | 21.8 |
| PH 3.1, room temperature for 4h | 2.4 | 97.6 | 0.0 | 30.2 | 45.6 | 24.2 |
| PH 8.5, room temperature for 4h | 2.5 | 97.5 | 0.1 | 27.7 | 47.8 | 24.5 |
| The sample was concentrated to 96.6 mg/ml | 2.5 | 97.5 | 0.1 | 35.2 | 41.1 | 23.7 |

The above results show that the anti-GITR antibody obtained by engineering modification in the present invention has good stability under the concentration of 20.3 mg/ml, repeated freezing and thawing, or under different temperatures and different pH conditions. And has good stability in the process of concentration to high concentration.

After the engineered fully human anti-GITR antibody was concentrated to 96.6 mg/ml, the stability of the sample was investigated. The results are shown in Table 24. No precipitation occurred during the concentration process, the appearance was clear and transparent, and the viscosity test result was 4.679 mPa · s.

**Table 24 Stability investigation results of high concentration samples**

| Sample Processing Information | SEC | | | CEX | | |
|---|---|---|---|---|---|---|
| | Polymer peak (%) | Monomer peak (%) | Degradation peak (%) | Acid peak (%) | Main peak (%) | Alkalin e peak (%) |
| The sample was concentrated to 96.6 mg/ml | 2.5 | 97.5 | 0.1 | 35.2 | 41.1 | 23.7 |

The above results show that the anti-GITR antibody obtained by engineering modification in the present invention has better stability under high concentration condition.

### Discussion

(1) Antibodies can be obtained by immunizing wild-type mice, but mouse antibodies need to be humanized to obtain humanized antibodies. The disadvantage is that the modified antibody may be more immunogenic and the structure of the antibody may be changed resulting in loss of activity or poor manufacturability.
(2) Fully human antibodies can be obtained by immunizing fully human transgenic mice, but the number or affinity of the obtained antibodies will be poor.
(3) Antibody expression and activity screening can be carried out by constructing immunized mouse antibody library with phage display technology, but the random recombination of antibody heavy and light chains will result in poor production of the formed antibodies.
(4) The antibody can be expressed and screened by phage display technology by constructing a human antibody library. But the affinity of the obtained antibody will be poor because it has not been immunized.
(5) The immunogen can be polypeptides, proteins, other types of cells and genes, but there will be problems such as incorrect conformation, low expression, and poor immunogenicity.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

### Sequence information of the present invention

**Table 28 Amino acid sequence number of the heavy and light chains of GITR antibody**

| Antibody number | Heavy chain | | | | Light chain | | | |
|---|---|---|---|---|---|---|---|---|
| | Variabl e region | VH -CDR1 | VH -CDR2 | VH -CDR3 | Variabl e region | VL -CDR1 | VL -CDR2 | VL -CDR3 |
| 1 (3503-mAb019) | 1 | 2 | 3 | 4 | 5 | 6 | 7 | 8 |
| 2 (3503-mAb070) | 9 | 10 | 11 | 12 | 13 | 14 | 15 | 16 |
| 3 (3503-mAb076) | 17 | 18 | 19 | 20 | 21 | 22 | 23 | 24 |
| 4 (3503-mAb064) | 25 | 26 | 27 | 28 | 29 | 30 | 31 | 32 |
| 5 (3503-hab019e1) | 41 | 2 | 3 | 4 | 5 | 6 | 7 | 8 |
| 6 (3503-hab019e2) | 42 | 2 | 3 | 43 | 5 | 6 | 7 | 8 |
| 7 (3503-hab019e3) | 44 | 2 | 3 | 45 | 5 | 6 | 7 | 8 |
| 8 (3503-hab070el) | 9 | 10 | 11 | 12 | 48 | 14 | 15 | 16 |
| 9 (3503-hab076el) | 17 | 18 | 19 | 20 | 49 | 22 | 23 | 24 |
| 10 (3503-hab064el) | 46 | 26 | 27 | 28 | 47 | 30 | 31 | 32 |

**Table 29 Nucleotide sequence number of the heavy and light chains of the GITR antibody**

| Antibody number | Clone number | Heavy chain variable region | Light chain variable region |
|---|---|---|---|
| 1 (3503-mAb019) | 96A10H9 | 33 | 34 |
| 2 (3503-mAb070) | 272G5E1 | 35 | 36 |
| 3 (3503-mAb076) | 277C12G4 | 37 | 38 |
| 4 (3503-mAb064) | 265G9A11 | 39 | 40 |
| 5 (3503-hab019e1) | 96A10H9 | 50 | 34 |
| 6 (3503-hab019e2) | | 51 | 34 |
| 7 (3503-hab019e3) | | 52 | 34 |
| 8 (3503-hab070e 1) | 272G5E1 | 35 | 55 |
| 9 (3503-hab076el) | 277C12G4 | 37 | 56 |
| 10 (3503-hab064e1) | 265G9A11 | 53 | 54 |

**Table 30 Sequence Information**

| SEQ ID NO. | Name | Sequence | Sequence Length |
|---|---|---|---|
| 1 | 3503-mAb019 VH | QVQLVESGGGVVQPGRSLRLSCAASGFTFSSYGMH WVRQAPGKGLEWVAVIWYAGSRKFYADSVEGRFTI SRDNSKNTLYLQMSSVRPEDTAVYYCARGGSLDGG FFYYGMDVWGQGTTVTVSS | 124aa |
| 2 | 3503-mAb019 VH-CDR1 | SYGMH | 5aa |
| 3 | 3503-mAb019 VH-CDR2 | VIWYAGSRKFYADSVEG | 17aa |
| 4 | 3503-mAb019 VH-CDR3 | GGSLDGGFFYYGMDV | 15aa |
| 5 | 3503-mAb019 VL | DIQMTQSPSTLSASVGDRVTITCRASQSISSWLAWYQ QKPGKAPKLLIYKASSLESGVPSRFSGSGSGTEFTLTI SSLQPDDFATYYCQQYNSYMYTFGQGTKLEIK | 107aa |
| 6 | 3503-mAb019 VL-CDR1 | RASQSISSWLA | 11aa |
| 7 | 3503-mAb019 VL-CDR2 | KASSLES | 7aa |
| 8 | 3503-mAb019 VL-CDR3 | QQYNSYMYT | 9aa |
| 9 | 3503-mAb070 VH | QVQLVESGGGVVQPGRSLRLSCAASGFTFSSYGMH WVRQAPGKGLEWVAVIWYETSNKFYVDSVKGRFTI SRDNSKNTLYLQMNSLRAEDTAVYYCARYGASSGW YWYFDLWGRGTLVTVSS | 122aa |
| 10 | 3503-mAb070 VH-CDR1 | SYGMH | 5aa |
| 11 | 3503-mAb070 VH-CDR2 | VIWYETSNKFYVDSVKG | 17aa |
| 12 | 3503-mAb070 VH-CDR3 | YGASSGWYWYFDL | 13aa |
| 13 | 3503-mAb070 VL | DIQLTQSPSFLSASVGDRVTITCRASQGISSFLAWYQ QKPGKAPKLLIYAASTLQSGVPSRFSGSGSGTEFTLTI SSLQPEDFATYCCQQLNSYPFTFGQGTKLEIK | 107aa |
| 14 | 3503-mAb070 VL-CDR1 | RASQGISSFLA | 11aa |
| 15 | 3503-mAb070 VL-CDR2 | AASTLQS | 7aa |
| 16 | 3503-mAb070 VL-CDR3 | QQLNSYPFT | 9aa |
| 17 | 3503-mAb076 VH | QVQLVESGGGVVQPGRSLRLSCAASGFTFSSYGMH WVRQAPGKGLEWVAVIWYETSNKYYADSVKGRFTI SRDNSKNTLYLQMNSLRAEDTAVYYCARYGASSGW YWYFDLWGRGTLVTVSS | 120aa |
| 18 | 3503-mAb076 VH-CDR1 | SYGMH | 5aa |
| 19 | 3503-mAb076 VH-CDR2 | VIWYETSNKYYADSVKG | 17aa |
| 20 | 3503-mAb076 VH-CDR3 | YGASSGWYWYFDL | 13aa |
| 21 | 3503-mAb076 VL | DIQLTQSPSFLSASVGDRVTITCRASQGISSYLAWYQ QKPGKAPKLLIYAASTLQSGVPSRFSGSGSGTEFTLTI SSLQPEDFATYCCQQLNSYPFTFGQGTKLEIK | 107aa |
| 22 | 3503-mAb076 VL-CDR1 | RASQGISSYLA | 11aa |
| 23 | 3503-mAb076 VL-CDR2 | AASTLQS | 7aa |
| 24 | 3503-mAb076 VL-CDR3 | QQLNSYPFT | 9aa |
| 25 | 3503-mAb064 VH | QVQLVESGGGVVQPGRSLRLSCAASGFTFSSYGMH WVRQAPGKGLEWVAVIWYETSNKYYADSVKGRFTI SRDNSKNTLYLQMNNLRAEDTAVYYCARYGGNSG WYWYFDLWGRGTLVTVSS | 122aa |
| 26 | 3503-mAb064 VH-CDR1 | SYGMH | 5aa |
| 27 | 3503-mAb064 VH-CDR2 | VIWYETSNKYYADSVKG | 17aa |
| 28 | 3503-mAb064 VH-CDR3 | YGGNSGWYWYFDL | 13aa |
| 29 | 3503-mAb064 VL | DIQLTQSPSFLSASVGDRVTITCRASQGISSFLAWYQ QKPGTAPKLLIYAASTLQSGVPSRFSGSGSGTEFTLTI SSLQPEDFATYCCQQLNSYPFTFGQGTKLEIK | 107aa |
| 30 | 3503-mAb064 VL-CDR1 | RASQGISSFLA | 11aa |
| 31 | 3503-mAb064 VL-CDR2 | AASTLQS | 7aa |
| 32 | 3503-mAb064 VL-CDR3 | QQLNSYPFT | 9aa |
| 33 | Nucleotide sequence of 3503-mAb019 VH | CAGGTGCAGCTGGTGGAGTCTGGGGGAGGCGTGGTC CAGCCTGGGAGGTCCCTGAGACTCTCCTGTGCAGCG TCTGGATTCACCTTCAGTAGCTATGGCATGCACTGGG TCCGCCAGGCTCCAGGCAAGGGGCTGGAGTGGGTGG CAGTTATTTGGTATGCTGGAAGTAGGAAGTTCTATGC AGACTCTGTGGAGGGCCGATTCACCATCTCCAGAGA CAATTCCAAGAACACGTTGTATCTGCAAATGAGCAG TGTCAGACCCGAGGACACGGCTGTGTATTACTGTGC GAGAGGGGGGTCCCTCGACGGTGGCTTCTTCTACTA CGGTATGGACGTCTGGGGCCAAGGGACCACGGTCAC CGTCTCCTCA | 372nt |
| 34 | Nucleotide sequence of 3503-mAb019 VL | GACATCCAGATGACCCAGTCTCCTTCCACCCTGTCTG CATCTGTAGGAGACAGAGTCACCATCACTTGCCGGG CCAGTCAGAGTATTAGTAGCTGGTTGGCCTGGTATCA GCAGAAACCAGGGAAAGCCCCTAAGCTCCTGATCTA TAAGGCGTCTAGTTTAGAAAGTGGGGTCCCATCAAG GTTCAGCGGCAGTGGATCTGGGACAGAATTCACTCT CACCATCAGCAGCCTGCAGCCTGATGATTTTGCAACT TATTACTGCCAACAGTATAATAGTTATATGTACACTT TTGGCCAGGGGACCAAGCTGGAGATCAAA | 321nt |
| 35 | Nucleotide sequence of 3503-mAb070 VH | CAGGTGCAGCTGGTGGAGTCTGGGGGAGGCGTGGTC CAGCCTGGGAGGTCCCTGAGACTCTCCTGTGCAGCG TCTGGATTCACCTTCAGTAGCTATGGCATGCACTGGG TCCGCCAGGCTCCAGGCAAGGGGCTGGAGTGGGTGG CAGTTATATGGTATGAAACAAGTAATAAATTCTATGT AGACTCCGTGAAGGGCCGATTCACCATCTCCAGAGA CAATTCCAAGAACACGCTGTATCTGCAAATGAACAG CCTGAGAGCCGAGGACACGGCTGTGTATTACTGTGC GAGATACGGGGCTAGCAGTGGCTGGTACTGGTACTT CGATCTCTGGGGCCGTGGCACCCTGGTCACTGTCTCC TCA | 366nt |
| 36 | Nucleotide sequence of 3503-mAb070 VL | GACATCCAGTTGACCCAGTCTCCATCCTTCCTGTCTG CATCTGTAGGAGACAGAGTCACCATCACTTGCCGGG CCAGTCAGGGCATTAGCAGTTTTTAGCCTGGTATCA GCAAAAACCAGGGAAAGCCCCTAAGCTCCTGATCTA TGCTGCATCCACTTTGCAAAGTGGGGTCCCATCAAG GTTCAGCGGCAGTGGCTCTGGGACAGAATTCACTCT CACAATCAGCAGCCTGCAGCCTGAAGATTTTGCAAC TTATTGCTGTCAACAGCTTAATAGTTACCCGTTCACT TTTGGCCAGGGGACCAAGCTGGAGATCAAA | 321nt |
| 37 | Nucleotide sequence of 3503-mAb076 VH | CAGGTGCAGCTGGTGGAGTCTGGGGGAGGCGTGGTC CAGCCTGGGAGGTCCCTGAGACTCTCCTGTGCAGCG TCTGGATTCACCTTCAGTAGTTATGGCATGCACTGGG TCCGCCAGGCTCCAGGCAAGGGGCTGGAGTGGGTGG CAGTTATATGGTATGAGACCAGTAATAAATACTATG CAGACTCCGTGAAGGGCCGATTCACCATCTCCAGAG ACAATTCCAAGAACACGCTGTATCTGCAAATGAACA GCCTGAGAGCCGAGGACACGGCTGTATATTACTGTG CGAGATACGGGGCTAGCAGTGGCTGGTACTGGTACT TCGATCTCTGGGGCCGCGGCACCCTGGTCACTGTCTC CTCA | 366nt |
| 38 | Nucleotide sequence of 3503-mAb076 VL | GACATCCAGTTGACCCAGTCTCCATCCTTCCTGTCTG CATCTGTAGGAGACAGAGTCACCATCACTTGCCGGG CCAGTCAGGGCATTAGCAGTTATTTAGCCTGGTATCA GCAAAAACCAGGGAAAGCCCCTAAGCTCCTGATCTA TGCTGCATCCACTTTGCAAAGTGGGGTCCCATCAAG GTTCAGCGGCAGTGGATCTGGGACAGAATTCACTCT CACAATCAGCAGCCTGCAGCCTGAAGATTTTGCAAC TTATTGCTGTCAACAGCTTAATAGTTACCCGTTCACT TTTGGCCAGGGGACCAAGCTGGAGATCAAA | 321nt |
| 39 | Nucleotide sequence of 3503-mAb064 VH | CAGGTGCAGCTGGTGGAGTCTGGGGGAGGCGTGGTC CAGCCTGGGAGGTCCCTGAGACTCTCCTGTGCAGCG TCTGGATTCACCTTCAGTAGTTATGGCATGCACTGGG TCCGCCAGGCTCCAGGCAAGGGGCTGGAGTGGGTGG CAGTTATATGGTATGAAACAAGTAATAAATACTATG CAGACTCCGTGAAGGGCCGATTCACCATCTCCAGAG ACAATTCCAAGAACACGCTGTATCTGCAAATGAACA ATTTGAGAGCCGAGGACACGGCTGTGTATTACTGTG CGAGATACGGGGGTAACAGTGGCTGGTACTGGTACT TCGATCTCTGGGGCCGTGGCACCCTGGTCACTGTCTC | 366nt |
| | | CTCA | |
| 40 | Nucleotide sequence of 3503-mAb064 VL | GACATCCAGTTGACCCAGTCTCCATCCTTCCTGTCTG CATCTGTAGGAGACAGAGTCACCATCACTTGCCGGG CCAGTCAGGGCATTAGCAGTTTTTTAGCCTGGTATCA GCAAAAACCAGGGACAGCCCCTAAGCTCCTGATCTA TGCTGCATCCACTTTGCAAAGTGGGGTCCCATCAAG GTTCAGCGGCAGTGGCTCTGGGACAGAATTCACTCT CACAATCAGCAGCCTGCAGCCTGAAGATTTTGCAAC TTATTGCTGTCAGCAGCTTAATAGTTACCCGTTCACT TTTGGCCAGGGGACCAAGCTGGAGATCAAA | 321nt |
| 41 | 3503-habO19e1 VH | QVQLVESGGGVVQPGRSLRLSCAASGFTFSSYGMH WVRQAPGKGLEWVAVIWYAGSRKFYADSVEGRFTI SRDNSKNTLYLQMNSLRAEDTAVYYCARGGSLDGG FFYYGMDVWGQGTTVTVSS | 124aa |
| 42 | 3503-hab019e2 VH | QVQLVESGGGVVQPGRSLRLSCAASGFTFSSYGMH WVRQAPGKGLEWVAVIWYAGSRKFYADSVEGRFTI SRDNSKNTLYLQMNSLRAEDTAVYYCARGGSLEGG FFYYGMDVWGQGTTVTVSS | 124aa |
| 43 | 3503-hab019e2 VH-CDR3 | GGSLEGGFFYYGMDV | 15aa |
| 44 | 3503-hab019e3 VH | QVQLVESGGGVVQPGRSLRLSCAASGFTFSSYGMH WVRQAPGKGLEWVAVIWYAGSRKFYADSVEGRFTI SRDNSKNTLYLQMNSLRAEDTAVYYCARGGSLDAG FFYYGMDVWGQGTTVTVSS | 124aa |
| 45 | 3503-hab019e3 VH-CDR3 | GGSLDAGFFYYGMDV | 15aa |
| 46 | 3503-hab064e1 VH | QVQLVESGGGVVQPGRSLRLSCAASGFTFSSYGMH WVRQAPGKGLEWVAVIWYETSNKYYADSVKGRFTI SRDNSKNTLYLQMNSLRAEDTAVYYCARYGGNSG WYWYFDLWGRGTLVTVSS | 122aa |
| 47 | 3503-hab064e1 VL | DIQLTQSPSFLSASVGDRVTITCRASQGISSFLAWYQ QKPGKAPKLLIYAASTLQSGVPSRFSGSGSGTEFTLTI SSLQPEDFATYYCQQLNSYPFTFGQGTKLEIK | 107aa |
| 48 | 3503-hab070e1 VL | DIQLTQSPSFLSASVGDRVTITCRASQGISSFLAWYQ QKPGKAPKLLIYAASTLQSGVPSRFSGSGSGTEFTLTI | 107aa |
| | | SSLQPEDFATYYCQQLNSYPFTFGQGTKLEIK | |
| 49 | 3503-hab076e1 VL | DIQLTQSPSFLSASVGDRVTITCRASQGISSYLAWYQ QKPGKAPKLLIYAASTLQSGVPSRFSGSGSGTEFTLTI SSLQPEDFATYYCQQLNSYPFTFGQGTKLEIK | 107aa |
| 50 | Nucleotide sequence of 3503-hab019e1 VH | CAGGTGCAGCTGGTGGAGAGCGGAGGCGGAGTGGT GCAGCCTGGCAGAAGCCTGAGACTGAGCTGCGCCGC CAGCGGCTTCACCTTCAGCTCCTACGGCATGCACTGG GTGAGGCAGGCCCCTGGAAAAGGCCTGGAGTGGGTG GCCGTGATCTGGTACGCCGGCAGCAGGAAGTTCTAC GCCGACTCCGTGGAGGGCAGATTCACCATCTCCAGG GACAACAGCAAGAACACCCTGTACCTGCAGATGAAC AGCCTGAGGGCCGAGGACACCGCCGTGTACTACTGC GCCAGAGGCGGAAGCCTGGACGGCGGCTTCTTCTAC TACGGCATGGATGTGTGGGGCCAGGGCACCACCGTG ACAGTGAGCAGC | 372nt |
| 51 | Nucleotide sequence of 3503-hab019e2 VH | CAGGTGCAGCTGGTGGAGAGCGGAGGCGGAGTGGT GCAGCCTGGCAGAAGCCTGAGACTGAGCTGCGCCGC CAGCGGCTTCACCTTCAGCTCCTACGGCATGCACTGG GTGAGGCAGGCCCCTGGAAAGGCCTGGAGTGGGTG GCCGTGATCTGGTACGCCGGCAGCAGGAAGTTCTAC GCCGACTCCGTGGAGGGCAGATTCACCATCTCCAGG GACAACAGCAAGAACACCCTGTACCTGCAGATGAAC AGCCTGAGGGCCGAGGACACCGCCGTGTACTACTGC GCCAGAGGCGGAAGCCTGGAGGGCGGCTTCTTCTAC TACGGCATGGATGTGTGGGGCCAGGGCACCACCGTG ACAGTGAGCAGC | 372nt |
| 52 | Nucleotide sequence of 3503-hab019e3 VH | CAGGTGCAGCTGGTGGAGAGCGGAGGCGGAGTGGT GCAGCCTGGCAGAAGCCTGAGACTGAGCTGCGCCGC CAGCGGCTTCACCTTCAGCTCCTACGGCATGCACTGG GTGAGGCAGGCCCCTGGAAAGGCCTGGAGTGGGTG GCCGTGATCTGGTACGCCGGCAGCAGGAAGTTCTAC GCCGACTCCGTGGAGGGCAGATTCACCATCTCCAGG GACAACAGCAAGAACACCCTGTACCTGCAGATGAAC AGCCTGAGGGCCGAGGACACCGCCGTGTACTACTGC GCCAGAGGCGGAAGCCTGGACGCCGGCTTCTTCTAC TACGGCATGGATGTGTGGGGCCAGGGCACCACCGTG ACAGTGAGCAGC | 372nt |
| 53 | Nucleotide sequence of 3503-hab064e1 VH | CAGGTGCAGCTGGTGGAGTCTGGGGGAGGCGTGGTC CAGCCTGGGAGGTCCCTGAGACTCTCCTGTGCAGCG TCTGGATTCACCTTCAGTAGTTATGGCATGCACTGGG TCCGCCAGGCTCCAGGCAAGGGGCTGGAGTGGGTGG CAGTTATATGGTATGAAACAAGTAATAAATACTATG CAGACTCCGTGAAGGGCCGATTCACCATCTCCAGAG ACAATTCCAAGAACACGCTGTATCTGCAAATGAACA GTTTGAGAGCCGAGGACACGGCTGTGTATTACTGTG CGAGATACGGGGGTAACAGTGGCTGGTACTGGTACT TCGATCTCTGGGGCCGTGGCACCCTGGTCACTGTCTC CTCA | 366nt |
| 54 | Nucleotide sequence of 3503-hab064e1 VL | GACATCCAGTTGACCCAGTCTCCATCCTTCCTGTCTG CATCTGTAGGAGACAGAGTCACCATCACTTGCCGGG CCAGTCAGGGCATTAGCAGTTTTTTAGCCTGGTATCA GCAAAAACCAGGGAAAGCCCCTAAGCTCCTGATCTA TGCTGCATCCACTTTGCAAAGTGGGGTCCCATCAAG GTTCAGCGGCAGTGGCTCTGGGACAGAATTCACTCT CACAATCAGCAGCCTGCAGCCTGAAGATTTTGCAAC TTATTACTGTCAGCAGCTTAATAGTTACCCGTTCACT TTTGGCCAGGGGACCAAGCTGGAGATCAAA | 321nt |
| 55 | Nucleotide sequence of 3503-hab070e1 VL | GACATCCAGTTGACCCAGTCTCCATCCTTCCTGTCTG CATCTGTAGGAGACAGAGTCACCATCACTTGCCGGG CCAGTCAGGGCATTAGCAGTTTTTAGCCTGGTATCA GCAAAAACCAGGGAAAGCCCCTAAGCTCCTGATCTA TGCTGCATCCACTTTGCAAAGTGGGGTCCCATCAAG GTTCAGCGGCAGTGGCTCTGGGACAGAATTCACTCT CACAATCAGCAGCCTGCAGCCTGAAGATTTTGCAAC TTATTACTGTCAACAGCTTAATAGTTACCCGTTCACT TTTGGCCAGGGGACCAAGCTGGAGATCAAA | 321nt |
| 56 | Nucleotide sequence of 3503-hab076e1 VL | GACATCCAGTTGACCCAGTCTCCATCCTTCCTGTCTG CATCTGTAGGAGACAGAGTCACCATCACTTGCCGGG CCAGTCAGGGCATTAGCAGTTATTTAGCCTGGTATCA GCAAAAACCAGGGAAAGCCCCTAAGCTCCTGATCTA TGCTGCATCCACTTTGCAAAGTGGGGTCCCATCAAG GTTCAGCGGCAGTGGATCTGGGACAGAATTCACTCT CACAATCAGCAGCCTGCAGCCTGAAGATTTTGCAAC TTATTACTGTCAACAGCTTAATAGTTACCCGTTCACT TTTGGCCAGGGGACCAAGCTGGAGATCAAA | 321nt |
| 57 | Human GITR extracellular domain Gln26-Glu161 (Thr45Ala) | QRPTGGPGCGPGRLLLGTGADARCCRVHTTRCCRDYP GEECCSEWDCMCVQPEFHCGDPCCTTCRHHPCPPGQG VQSQGKFSFGFQCIDCASGTFSGGHEGHCKPWTDCTQF GFLTVFPGNKTHNAVCVPGSPPAE | 136aa |
| 58 | Human GITR full length | ATGGCACAGCACGGGGCGATGGGCGCGTTTCGGGCC CTGTGCGGCCTGGCGCTGCTGTGCGCGCTCAGCCTG GGTCAGCGCCCCACCGGGGGTCCCGGGTGCGGCCCT GGGCGCCTCCTGCTTGGGACGGAACGGACGCGCGC TGCTGCCGGGTTCACACGACGCGCTGCTGCCGCGAT TACCCGGGCGAGGAGTGCTGTTCCGAGTGGGACTGC ATGTGTGTCCAGCCTGAATTCCACTGCGGAGACCCTT GCTGCACGACCTGCCGGCACCACCCTTGTCCCCCAG GCCAGGGGGTACAGTCCCAGGGGAAATTCAGTTTTG GCTTCCAGTGTATCGACTGTGCCTCGGGGACCTTCTC CGGGGGCCACGAAGGCCACTGCAAACCTTGGACAGA CTGCACCCAGTTCGGGTTTCTCACTGTGTTCCCTGGG AACAAGACCCACAACGCTGTGTGCGTCCCAGGGTCC CCGCCGGCAGAGCCGCTTGGGTGGCTGACCGTCGTC CTCCTGGCCGTGGCCGCCTGCGTCCTCCTCCTGACCT CGGCCCAGCTTGGACTGCACATCTGGCAGCTGAGGA | 726nt |
| | | GTCAGTGCATGTGGCCCCGAGAGACCCAGCTGCTGC TGGAGGTGCCGCCGTCGACCGAAGACGCCAGAAGCT GCCAGTTCCCCGAGGAAGAGCGGGGCGAGCGATCGG CAGAGGAGAAGGGGCGGCTGGGAGACCTGTGGGTG TGA | |

| | | | |
|---|---|---|---|
| Note: CDR are divided by Kabat method. | | | |

## Claims

1. A heavy chain variable region of an antibody, wherein the heavy chain variable region comprises complementary determining regions or CDRs selected from the group consisting of:
VH-CDR1 shown in SEQ ID NO: 8n+2,
VH-CDR2 shown in SEQ ID NO: 8n+3, and
VH-CDR3 shown in SEQ ID NO: 8n+4;
wherein, each n is independently 0, 1, 2 or 3;
wherein, any one of the above amino acid sequences also includes a derivative sequence that is optionally with at least one amino acid added, deleted, modified, and/or substituted, and is capable of retaining the binding affinity to GITR.

2. A heavy chain of an antibody, wherein the heavy chain comprises the heavy chain variable region of claim 1.

3. A light chain variable region of an antibody, wherein the light chain variable region comprises complementary determining regions or CDRs selected from the group consisting of:
VL-CDR1 shown in SEQ ID NO: 8n+6,
VL-CDR2 shown in SEQ ID NO: 8n+7, and
VL-CDR3 shown in SEQ ID NO: 8n+8;
wherein, each n is independently 0, 1, 2 or 3;
wherein, any one of the above amino acid sequences also includes a derivative sequence that is optionally with at least one amino acid added, deleted, modified, and/or substituted, and is capable of retaining the binding affinity to GITR.

4. A light chain of an antibody, wherein the light chain comprises the light chain variable region of claim 3.

5. An antibody, wherein the antibody comprises:
(1) the heavy chain variable region of claim 1; and/or
(2) the light chain variable region of claim 3;
or the antibody comprises: the heavy chain of claim 2; and/or the light chain of claim 4,
wherein, any one of the above amino acid sequences also includes a derivative sequence that is optionally with at least one amino acid added, deleted, modified, and/or substituted, and is capable of retaining the binding affinity to GITR.

6. The antibody of claim 5, wherein the antibody comprises the heavy chain variable region of claim 1 and the light chain variable region of claim 3;
wherein, the heavy chain variable region and the light chain variable region comprise CDRs selected from the group consisting of:
| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 2 | 3 | 4, 43, 45 | 6 | 7 | 8 |
| 10 | 11 | 12 | 14 | 15 | 16 |
| 18 | 19 | 20 | 22 | 23 | 24 |
| 26 | 27 | 28 | 30 | 31 | 32 |
wherein, any one of the above amino acid sequences also includes a derivative sequence that is optionally with at least one amino acid added, deleted, modified, and/or substituted, and is capable of retaining the binding affinity to GITR.

7. The antibody of claim 5, wherein the heavy chain variable region of the antibody comprises the amino acid sequences shown in SEQ ID NO: 1, 9, 17, 25, 41, 42, 44 or 46; and/or the light chain variable region of the antibody contains the amino acid sequences shown in SEQ ID NO: 5, 13, 21, 29, 47, 48 or 49.

8. The antibody of claim 6, wherein the antibody is selected from the group consisting of:
| Antibody number | Clone | VH sequence number | VL sequence number |
|---|---|---|---|
| 1 (3503-mAb019) | 96A10H9 | 1 | 5 |
| 2 (3503-mAb070) | 272G5E1 | 9 | 13 |
| 3 (3503-mAb076) | 277C12G4 | 17 | 21 |
| 4 (3503-mAb064) | 265G9A11 | 25 | 29 |
| 5 (3503-hab019e1) | 96A10H9 | 41 | 5 |
| 6 (3503-hab019e2) | 96A10H9 | 42 | 5 |
| 7 (3503-hab019e3) | 96A10H9 | 44 | 5 |
| 8 (3503-hab070e1) | 272G5E1 | 9 | 48 |
| 9 (3503-hab076e1) | 277C12G4 | 17 | 49 |
| 10 (3503-hab064e1) | 265G9A11 | 46 | 47. |

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence that assists expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or the antibody of any one of claims 5-8; and
(2) the recombinant protein of claim 9.

11. The polynucleotide of claim 10, wherein the polynucleotide encoding the heavy chain variable region is shown in SEQ ID NO: 33, 35, 37, 39, 50, 51, 52 or 53; and/or, the polynucleotide encoding the light chain variable region is shown in SEQ ID NO: 34, 36, 38, 40, 54, 55 or 56.

12. The polynucleotide of claim 11, wherein the polynucleotide encoding the heavy chain variable region and the polynucleotide encoding the light chain variable region are selected from the group consisting of:
| Clone | Sequence number of the polynucleotide encoding VH | Sequence number of the polynucleotide encoding VL |
|---|---|---|
| 1 (3503-mAb019) | 33 | 34 |
| 2 (3503-mAb070) | 35 | 36 |
| 3 (3503-mAb076) | 37 | 38 |
| 4 (3503-mAb064) | 39 | 40 |
| 5 (3503-hab019e1) | 50 | 34 |
| 6 (3503-hab019e2) | 51 | 34 |
| 7 (3503-hab019e3) | 52 | 34 |
| 8 (3503-hab070e1) | 35 | 55 |
| 9 (3503-hab076e1) | 37 | 56 |
| 10 (3503-hab064e1) | 53 | 54. |

13. A vector comprising the polynucleotide according to any one of claims 10 to 12 of the present invention.

14. A genetically engineered host cell, wherein the host cell contains the vector of claim 13 or the genome thereof is integrated with the polynucleotide of any one of claims 10-12.

15. An antibody conjugate, wherein the antibody conjugate comprises:
(a) an antibody moiety, which is selected from the group consisting of: the heavy chain variable region of claim 1, the heavy chain of claim 2, the light chain variable region of claim 3, the light chain of claim 4, the antibody of any one of claims 5-8, and a combination thereof; and
(b) a coupling moiety coupled to the antibody moiety, and the coupling moiety is selected from the group consisting of a detectable label, a drug, a toxin, a cytokine, a radionuclide, an enzyme, and a combination thereof.

16. An immune cell, which expresses or is exposed outside the cell membrane with the antibody of any one of claims 5-8.

17. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, the antibody conjugate of claim 15, the immune cell of claim 16, and combinations thereof; and
(ii) a pharmaceutically acceptable carrier.


SEQUENCE LISTING

<110>  SHANGHAI PHARMAEXPLORER CO., LTD.
       PHARMAEXPLORER LIMITED

<120>  Fully humanized anti-GITR antibody and preparation method therefor

<130>  P2018-0892

<150>  CN201811444696.1
<151>  2018-11-29

<160>  58

<170>  PatentIn version 3.5

<210>  1
<211>  124
<212>  PRT
<213>  Artificial sequence

<220>
<223>  Heavy chain variable region

<400>  1

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Ala Gly Ser Arg Lys Phe Tyr Ala Asp Ser Val
    50                  55                  60

Glu Gly Arg Phe Thr Ile Ser Arg Asp Asn Ser Lys Asn Thr Leu Tyr
65                  70                  75                  80

Leu Gln Met Ser Ser Val Arg Pro Glu Asp Thr Ala Val Tyr Tyr Cys
                85                  90                  95

Ala Arg Gly Gly Ser Leu Asp Gly Gly Phe Phe Tyr Tyr Gly Met Asp
            100                 105                 110

Val Trp Gly Gln Gly Thr Thr Val Thr Val Ser Ser
        115                 120

<210>  2
<211>  5
<212>  PRT

<213>  Artificial sequence

<220>
<223>   CDR1 of heavy chain


<400>  2

Ser Tyr Gly Met His
1               5


<210>  3
<211>  17
<212>  PRT
<213>  Artificial sequence

<220>
<223>   CDR2 of heavy chain


<400>  3

Val Ile Trp Tyr Ala Gly Ser Arg Lys Phe Tyr Ala Asp Ser Val Glu
1               5                   10                  15


Gly




<210>  4
<211>  15
<212>  PRT
<213>  Artificial sequence

<220>
<223>  CDR3  of heavy chain


<400>  4

Gly Gly Ser Leu Asp Gly Gly Phe Phe Tyr Tyr Gly Met Asp Val
1               5                   10                  15


<210>  5
<211>  107
<212>  PRT
<213>  Artificial sequence

<220>
<223>  Light chain variable region


<400>  5

Asp Ile Gln Met Thr Gln Ser Pro Ser Thr Leu Ser Ala Ser Val Gly
1               5                   10                  15


Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Met Tyr
                85                  90                  95

Thr Phe Gly Gln Gly Thr Lys Leu Glu Ile Lys
            100                 105

<210>  6
<211>  11
<212>  PRT
<213>  Artificial sequence

<220>
<223>   CDR1 of light chain

<400>  6

Arg Ala Ser Gln Ser Ile Ser Ser Trp Leu Ala
1               5                   10

<210>  7
<211>  7
<212>  PRT
<213>  Artificial sequence

<220>
<223>   CDR2 of light chain

<400>  7

Lys Ala Ser Ser Leu Glu Ser
1               5

<210>  8
<211>  9
<212>  PRT
<213>  Artificial sequence

<220>
<223>   CDR3 of light chain

<400>  8

Gln Gln Tyr Asn Ser Tyr Met Tyr Thr
1               5

<210>  9
<211>  122
<212>  PRT
<213>  Artificial sequence

<220>
<223>  Heavy chain variable region

<400>  9

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Glu Thr Ser Asn Lys Phe Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Gly Ala Ser Ser Gly Trp Tyr Trp Tyr Phe Asp Leu Trp
            100                 105                 110

Gly Arg Gly Thr Leu Val Thr Val Ser Ser
        115                 120

<210>  10
<211>  5
<212>  PRT
<213>  Artificial sequence

<220>
<223>   CDR1 of heavy chain

<400>  10

Ser Tyr Gly Met His
1               5

<210>  11

<211>  17
<212>  PRT
<213>  Artificial sequence

<220>
<223>   CDR2 of heavy chain


<400>  11

Val Ile Trp Tyr Glu Thr Ser Asn Lys Phe Tyr Val Asp Ser Val Lys
1               5                   10                  15


Gly




<210>  12
<211>  13
<212>  PRT
<213>  Artificial sequence

<220>
<223>   CDR3 of heavy chain


<400>  12

Tyr Gly Ala Ser Ser Gly Trp Tyr Trp Tyr Phe Asp Leu
1               5                   10


<210>  13
<211>  107
<212>  PRT
<213>  Artificial sequence

<220>
<223>  Light chain variable region


<400>  13

Asp Ile Gln Leu Thr Gln Ser Pro Ser Phe Leu Ser Ala Ser Val Gly
1               5                   10                  15


Asp Arg Val Thr Ile Thr Cys Arg Ala Ser Gln Gly Ile Ser Ser Phe
            20                  25                  30


Leu Ala Trp Tyr Gln Gln Lys Pro Gly Lys Ala Pro Lys Leu Leu Ile
        35                  40                  45


Tyr Ala Ala Ser Thr Leu Gln Ser Gly Val Pro Ser Arg Phe Ser Gly
    50                  55                  60


Ser Gly Ser Gly Thr Glu Phe Thr Leu Thr Ile Ser Ser Leu Gln Pro
65                  70                  75                  80

Glu Asp Phe Ala Thr Tyr Cys Cys Gln Gln Leu Asn Ser Tyr Pro Phe
                85                  90                  95

Thr Phe Gly Gln Gly Thr Lys Leu Glu Ile Lys
            100                 105

<210>  14
<211>  11
<212>  PRT
<213>  Artificial sequence

<220>
<223>  CDR1  of light chain

<400>  14

Arg Ala Ser Gln Gly Ile Ser Ser Phe Leu Ala
1               5                   10

<210>  15
<211>  7
<212>  PRT
<213>  Artificial sequence

<220>
<223>  CDR2 of light chain

<400>  15

Ala Ala Ser Thr Leu Gln Ser
1               5

<210>  16
<211>  9
<212>  PRT
<213>  Artificial sequence

<220>
<223>   CDR3 of light chain

<400>  16

Gln Gln Leu Asn Ser Tyr Pro Phe Thr
1               5

<210>  17
<211>  122
<212>  PRT
<213>  Artificial sequence

<220>
<223>  Heavy chain variable region

<400>  17

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Glu Thr Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Gly Ala Ser Ser Gly Trp Tyr Trp Tyr Phe Asp Leu Trp
            100                 105                 110

Gly Arg Gly Thr Leu Val Thr Val Ser Ser
        115                 120


<210>  18
<211>  5
<212>  PRT
<213>  Artificial sequence

<220>
<223>   CDR1 of heavy chain


<400>  18

Ser Tyr Gly Met His
1               5


<210>  19
<211>  17
<212>  PRT
<213>  Artificial sequence

<220>
<223>   CDR2 of heavy chain


<400>  19

Val Ile Trp Tyr Glu Thr Ser Asn Lys Tyr Tyr Ala Asp Ser Val Lys
1               5                   10                  15

Gly

<210>  20
<211>  13
<212>  PRT
<213>  Artificial sequence

<220>
<223>  CDR3  of heavy chain

<400>  20

Tyr Gly Ala Ser Ser Gly Trp Tyr Trp Tyr Phe Asp Leu
1               5                   10

<210>  21
<211>  107
<212>  PRT
<213>  Artificial sequence

<220>
<223>  Light chain variable region

<400>  21

Asp Ile Gln Leu Thr Gln Ser Pro Ser Phe Leu Ser Ala Ser Val Gly
1               5                   10                  15

Asp Arg Val Thr Ile Thr Cys Arg Ala Ser Gln Gly Ile Ser Ser Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Cys Cys Gln Gln Leu Asn Ser Tyr Pro Phe
                85                  90                  95

Thr Phe Gly Gln Gly Thr Lys Leu Glu Ile Lys
            100                 105

<210>  22
<211>  11
<212>  PRT
<213>  Artificial sequence

<220>
<223>   CDR1 of light chain


<400>  22

Arg Ala Ser Gln Gly Ile Ser Ser Tyr Leu Ala
1               5                   10


<210>  23
<211>  7
<212>  PRT
<213>  Artificial sequence

<220>
<223>   CDR2 of light chain


<400>  23

Ala Ala Ser Thr Leu Gln Ser
1               5


<210>  24
<211>  9
<212>  PRT
<213>  Artificial sequence

<220>
<223>   CDR3 of light chain


<400>  24

Gln Gln Leu Asn Ser Tyr Pro Phe Thr
1               5


<210>  25
<211>  122
<212>  PRT
<213>  Artificial sequence

<220>
<223>  Heavy chain variable region


<400>  25

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45

Ala Val Ile Trp Tyr Glu Thr Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Asn Leu Arg Ala Glu Asp Thr Ala Val Tyr Tyr Cys
                85                  90                  95

Ala Arg Tyr Gly Gly Asn Ser Gly Trp Tyr Trp Tyr Phe Asp Leu Trp
            100                 105                 110

Gly Arg Gly Thr Leu Val Thr Val Ser Ser
        115                 120

<210>  26
<211>  5
<212>  PRT
<213>  Artificial sequence

<220>
<223>   CDR1 of heavy chain

<400>  26

Ser Tyr Gly Met His
1               5

<210>  27
<211>  17
<212>  PRT
<213>  Artificial sequence

<220>
<223>   CDR2 of heavy chain

<400>  27

Val Ile Trp Tyr Glu Thr Ser Asn Lys Tyr Tyr Ala Asp Ser Val Lys
1               5                   10                  15

Gly

<210>  28
<211>  13
<212>  PRT
<213>  Artificial sequence

<220>
<223>  CDR3 of heavy chain

<400>  28

Tyr Gly Gly Asn Ser Gly Trp Tyr Trp Tyr Phe Asp Leu
1               5                   10


<210>  29
<211>  107
<212>  PRT
<213>  Artificial sequence

<220>
<223>  Light chain variable region


<400>  29

Asp Ile Gln Leu Thr Gln Ser Pro Ser Phe Leu Ser Ala Ser Val Gly
1               5                   10                  15


Asp Arg Val Thr Ile Thr Cys Arg Ala Ser Gln Gly Ile Ser Ser Phe
            20                  25                  30


Leu Ala Trp Tyr Gln Gln Lys Pro Gly Thr Ala Pro Lys Leu Leu Ile
        35                  40                  45


Tyr Ala Ala Ser Thr Leu Gln Ser Gly Val Pro Ser Arg Phe Ser Gly
    50                  55                  60


Ser Gly Ser Gly Thr Glu Phe Thr Leu Thr Ile Ser Ser Leu Gln Pro
65                  70                  75                  80


Glu Asp Phe Ala Thr Tyr Cys Cys Gln Gln Leu Asn Ser Tyr Pro Phe
                85                  90                  95


Thr Phe Gly Gln Gly Thr Lys Leu Glu Ile Lys
            100                 105


<210>  30
<211>  11
<212>  PRT
<213>  Artificial sequence

<220>
<223>   CDR1 of light chain


<400>  30

Arg Ala Ser Gln Gly Ile Ser Ser Phe Leu Ala
1               5                   10


<210>  31
<211>  7

<212>  PRT
<213>  Artificial sequence

<220>
<223>   CDR2 of light chain


<400>  31

Ala Ala Ser Thr Leu Gln Ser
1               5


<210>  32
<211>  9
<212>  PRT
<213>  Artificial sequence

<220>
<223>   CDR3 of light chain


<400>  32

Gln Gln Leu Asn Ser Tyr Pro Phe Thr
1               5


<210>  33
<211>  372
<212>  DNA
<213>  Artificial sequence

<220>
<223>  Heavy chain variable region


<400>  33
caggtgcagc tggtggagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atttggtatg ctggaagtag gaagttctat     180

gcagactctg tggagggccg attcaccatc tccagagaca attccaagaa cacgttgtat     240

ctgcaaatga gcagtgtcag acccgaggac acggctgtgt attactgtgc gagagggggg      300

tccctcgacg gtggcttctt ctactacggt atggacgtct ggggccaagg gaccacggtc     360

accgtctcct ca                                                         372


<210>  34
<211>  321
<212>  DNA
<213>  Artificial sequence

<220>
<223>  Light chain variable region


<400>  34

gacatccaga tgacccagtc tccttccacc ctgtctgcat ctgtaggaga cagagtcacc 60

atcacttgcc gggccagtca gagtattagt agctggttgg cctggtatca gcagaaacca 120

gggaaagccc ctaagctcct gatctataag gcgtctagtt tagaaagtgg ggtcccatca 180

aggttcagcg gcagtggatc tgggacagaa ttcactctca ccatcagcag cctgcagcct 240

gatgattttg caacttatta ctgccaacag tataatagtt atatgtacac ttttggccag 300

gggaccaagc tggagatcaa a 321

<210> 35
<211> 366
<212> DNA
<213> Artificial sequence

<220>
<223> Heavy chain variable region

<400> 35
caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatggtatg aaacaagtaa taaattctat 180

gtagactccg tgaagggccg attcaccatc tccagagaca attccaagaa cacgctgtat 240

ctgcaaatga acagcctgag agccgaggac acggctgtgt attactgtgc gagatacggg 300

gctagcagtg gctggtactg gtacttcgat ctctggggcc gtggcaccct ggtcactgtc 360

tcctca 366

<210> 36
<211> 321
<212> DNA
<213> Artificial sequence

<220>
<223> Light chain variable region

<400> 36
gacatccagt tgacccagtc tccatccttc ctgtctgcat ctgtaggaga cagagtcacc 60

atcacttgcc gggccagtca gggcattagc agttttttag cctggtatca gcaaaaacca 120

gggaaagccc ctaagctcct gatctatgct gcatccactt tgcaaagtgg ggtcccatca 180

aggttcagcg gcagtggctc tgggacagaa ttcactctca caatcagcag cctgcagcct 240

gaagattttg caacttattg ctgtcaacag cttaatagtt acccgttcac ttttggccag 300

gggaccaagc tggagatcaa a 321

<210> 37
<211> 366

<212>  DNA
<213>  Artificial sequence

<220>
<223>  Heavy chain variable region


<400>  37
caggtgcagc tggtggagtc tgggggaggc gtggtccagc ctgggaggtc cctgagactc      60

tcctgtgcag cgtctggatt caccttcagt agttatggca tgcactgggt ccgccaggct     120

ccaggcaagg ggctggagtg ggtggcagtt atatggtatg agaccagtaa taaatactat     180

gcagactccg tgaagggccg attcaccatc tccagagaca attccaagaa cacgctgtat     240

ctgcaaatga acagcctgag agccgaggac acggctgtat attactgtgc gagatacggg     300

gctagcagtg gctggtactg gtacttcgat ctctggggcc gcggcaccct ggtcactgtc     360

tcctca                                                                366


<210>  38
<211>  321
<212>  DNA
<213>  Artificial sequence

<220>
<223>  Light chain variable region


<400>  38
gacatccagt tgacccagtc tccatccttc ctgtctgcat ctgtaggaga cagagtcacc      60

atcacttgcc gggccagtca gggcattagc agttatttag cctggtatca gcaaaaacca     120

gggaaagccc ctaagctcct gatctatgct gcatccactt tgcaaagtgg ggtcccatca     180

aggttcagcg gcagtggatc tgggacagaa ttcactctca caatcagcag cctgcagcct     240

gaagattttg caacttattg ctgtcaacag cttaatagtt acccgttcac ttttggccag     300

gggaccaagc tggagatcaa a                                               321


<210>  39
<211>  366
<212>  DNA
<213>  Artificial sequence

<220>
<223>  Heavy chain variable region


<400>  39
caggtgcagc tggtggagtc tgggggaggc gtggtccagc ctgggaggtc cctgagactc      60

tcctgtgcag cgtctggatt caccttcagt agttatggca tgcactgggt ccgccaggct     120

ccaggcaagg ggctggagtg ggtggcagtt atatggtatg aaacaagtaa taaatactat     180

gcagactccg tgaagggccg attcaccatc tccagagaca attccaagaa cacgctgtat     240

ctgcaaatga acaatttgag agccgaggac acggctgtgt attactgtgc gagatacggg 300

ggtaacagtg gctggtactg gtacttcgat ctctggggcc gtggcaccct ggtcactgtc 360

tcctca 366

<210> 40
<211> 321
<212> DNA
<213> Artificial sequence

<220>
<223> Light chain variable region

<400> 40
gacatccagt tgacccagtc tccatccttc ctgtctgcat ctgtaggaga cagagtcacc 60

atcacttgcc gggccagtca gggcattagc agttttttag cctggtatca gcaaaaacca 120

gggacagccc ctaagctcct gatctatgct gcatccactt tgcaaagtgg ggtcccatca 180

aggttcagcg gcagtggctc tgggacagaa ttcactctca caatcagcag cctgcagcct 240

gaagattttg caacttattg ctgtcagcag cttaatagtt acccgttcac ttttggccag 300

gggaccaagc tggagatcaa a 321

<210> 41
<211> 124
<212> PRT
<213> Artificial sequence

<220>
<223> Heavy chain variable region

<400> 41

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Ala Gly Ser Arg Lys Phe Tyr Ala Asp Ser Val
    50                  55                  60

Glu Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85                  90                  95

Ala Arg Gly Gly Ser Leu Asp Gly Gly Phe Phe Tyr Tyr Gly Met Asp
            100                 105                 110

Val Trp Gly Gln Gly Thr Thr Val Thr Val Ser Ser
        115                 120

<210>  42
<211>  124
<212>  PRT
<213>  Artificial sequence

<220>
<223>  Heavy chain variable region

<400>  42

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Ala Gly Ser Arg Lys Phe Tyr Ala Asp Ser Val
    50                  55                  60

Glu Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Ser Leu Glu Gly Gly Phe Phe Tyr Tyr Gly Met Asp
            100                 105                 110

Val Trp Gly Gln Gly Thr Thr Val Thr Val Ser Ser
        115                 120

<210>  43
<211>  15
<212>  PRT
<213>  Artificial sequence

<220>
<223>  Heavy chain variable region

<400>  43

Gly Gly Ser Leu Glu Gly Gly Phe Phe Tyr Tyr Gly Met Asp Val
1               5                   10                  15


<210>  44
<211>  124
<212>  PRT
<213>  Artificial sequence

<220>
<223>  Heavy chain variable region


<400>  44

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Val Ile Trp Tyr Ala Gly Ser Arg Lys Phe Tyr Ala Asp Ser Val
    50                  55                  60


Glu Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Gly Gly Ser Leu Asp Ala Gly Phe Phe Tyr Tyr Gly Met Asp
            100                 105                 110


Val Trp Gly Gln Gly Thr Thr Val Thr Val Ser Ser
        115                 120


<210>  45
<211>  15
<212>  PRT
<213>  Artificial sequence

<220>
<223>  Heavy chain variable region


<400>  45

Gly Gly Ser Leu Asp Ala Gly Phe Phe Tyr Tyr Gly Met Asp Val
1               5                   10                  15

<210>  46
<211>  122
<212>  PRT
<213>  Artificial sequence

<220>
<223>  Heavy chain variable region


<400>  46

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Val Ile Trp Tyr Glu Thr Ser Asn Lys Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Tyr Gly Gly Asn Ser Gly Trp Tyr Trp Tyr Phe Asp Leu Trp
            100                 105                 110


Gly Arg Gly Thr Leu Val Thr Val Ser Ser
        115                 120


<210>  47
<211>  107
<212>  PRT
<213>  Artificial sequence

<220>
<223>  Light chain variable region


<400>  47

Asp Ile Gln Leu Thr Gln Ser Pro Ser Phe Leu Ser Ala Ser Val Gly
1               5                   10                  15


Asp Arg Val Thr Ile Thr Cys Arg Ala Ser Gln Gly Ile Ser Ser Phe
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Leu Asn Ser Tyr Pro Phe
                85                  90                  95

Thr Phe Gly Gln Gly Thr Lys Leu Glu Ile Lys
            100                 105

<210>  48
<211>  107
<212>  PRT
<213>  Artificial sequence

<220>
<223>  Light chain variable region

<400>  48

Asp Ile Gln Leu Thr Gln Ser Pro Ser Phe Leu Ser Ala Ser Val Gly
1               5                   10                  15

Asp Arg Val Thr Ile Thr Cys Arg Ala Ser Gln Gly Ile Ser Ser Phe
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Leu Asn Ser Tyr Pro Phe
                85                  90                  95

Thr Phe Gly Gln Gly Thr Lys Leu Glu Ile Lys
            100                 105

<210>  49
<211>  107
<212>  PRT
<213>  Artificial sequence

<220>
<223>  Light chain variable region

<400>  49

Asp Ile Gln Leu Thr Gln Ser Pro Ser Phe Leu Ser Ala Ser Val Gly
1               5                   10                  15

Asp Arg Val Thr Ile Thr Cys Arg Ala Ser Gln Gly Ile Ser Ser Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Leu Asn Ser Tyr Pro Phe
                85                  90                  95

Thr Phe Gly Gln Gly Thr Lys Leu Glu Ile Lys
            100                 105

<210>  50
<211>  372
<212>  DNA
<213>  Artificial sequence

<220>
<223>  Heavy chain variable region

<400>  50
caggtgcagc tggtggagag cggaggcgga gtggtgcagc ctggcagaag cctgagactg      60

agctgcgccg ccagcggctt caccttcagc tcctacggca tgcactgggt gaggcaggcc     120

cctggaaaag gcctggagtg ggtggccgtg atctggtacg ccggcagcag gaagttctac     180

gccgactccg tggagggcag attcaccatc tccagggaca acagcaagaa caccctgtac     240

ctgcagatga acagcctgag ggccgaggac accgccgtgt actactgcgc cagaggcgga     300

agcctggacg gcggcttctt ctactacggc atggatgtgt ggggccaggg caccaccgtg     360

acagtgagca gc                                                         372

<210>  51
<211>  372
<212>  DNA

<213>  Artificial sequence

<220>
<223>  Heavy chain variable region


<400>  51
caggtgcagc tggtggagag cggaggcgga gtggtgcagc ctggcagaag cctgagactg      60

agctgcgccg ccagcggctt caccttcagc tcctacggca tgcactgggt gaggcaggcc     120

cctggaaaag gcctggagtg ggtggccgtg atctggtacg ccggcagcag gaagttctac     180

gccgactccg tggagggcag attcaccatc tccagggaca acagcaagaa caccctgtac     240

ctgcagatga acagcctgag ggccgaggac accgccgtgt actactgcgc cagaggcgga     300

agcctggagg gcggcttctt ctactacggc atggatgtgt ggggccaggg caccaccgtg     360

acagtgagca gc                                                         372


<210>  52
<211>  372
<212>  DNA
<213>  Artificial sequence

<220>
<223>  Heavy chain variable region


<400>  52
caggtgcagc tggtggagag cggaggcgga gtggtgcagc ctggcagaag cctgagactg      60

agctgcgccg ccagcggctt caccttcagc tcctacggca tgcactgggt gaggcaggcc     120

cctggaaaag gcctggagtg ggtggccgtg atctggtacg ccggcagcag gaagttctac     180

gccgactccg tggagggcag attcaccatc tccagggaca acagcaagaa caccctgtac     240

ctgcagatga acagcctgag ggccgaggac accgccgtgt actactgcgc cagaggcgga     300

agcctggacg ccggcttctt ctactacggc atggatgtgt ggggccaggg caccaccgtg     360

acagtgagca gc                                                         372


<210>  53
<211>  366
<212>  DNA
<213>  Artificial sequence

<220>
<223>  Heavy chain variable region


<400>  53
caggtgcagc tggtggagtc tgggggaggc gtggtccagc ctgggaggtc cctgagactc      60

tcctgtgcag cgtctggatt caccttcagt agttatggca tgcactgggt ccgccaggct     120

ccaggcaagg ggctggagtg ggtggcagtt atatggtatg aaacaagtaa taaatactat     180

gcagactccg tgaagggccg attcaccatc tccagagaca attccaagaa cacgctgtat 240

ctgcaaatga acagtttgag agccgaggac acggctgtgt attactgtgc gagatacggg 300

ggtaacagtg gctggtactg gtacttcgat ctctggggcc gtggcaccct ggtcactgtc 360

tcctca 366

<210> 54
<211> 321
<212> DNA
<213> Artificial sequence

<220>
<223> Light chain variable region

<400> 54
gacatccagt tgacccagtc tccatccttc ctgtctgcat ctgtaggaga cagagtcacc 60

atcacttgcc gggccagtca gggcattagc agttttttag cctggtatca gcaaaaacca 120

gggaaagccc ctaagctcct gatctatgct gcatccactt tgcaaagtgg ggtcccatca 180

aggttcagcg gcagtggctc tgggacagaa ttcactctca caatcagcag cctgcagcct 240

gaagattttg caacttatta ctgtcagcag cttaatagtt acccgttcac ttttggccag 300

gggaccaagc tggagatcaa a 321

<210> 55
<211> 321
<212> DNA
<213> Artificial sequence

<220>
<223> Light chain variable region

<400> 55
gacatccagt tgacccagtc tccatccttc ctgtctgcat ctgtaggaga cagagtcacc 60

atcacttgcc gggccagtca gggcattagc agttttttag cctggtatca gcaaaaacca 120

gggaaagccc ctaagctcct gatctatgct gcatccactt tgcaaagtgg ggtcccatca 180

aggttcagcg gcagtggctc tgggacagaa ttcactctca caatcagcag cctgcagcct 240

gaagattttg caacttatta ctgtcaacag cttaatagtt acccgttcac ttttggccag 300

gggaccaagc tggagatcaa a 321

<210> 56
<211> 321
<212> DNA
<213> Artificial sequence

<220>
<223> Light chain variable region

<400>  56
gacatccagt tgacccagtc tccatccttc ctgtctgcat ctgtaggaga cagagtcacc      60

atcacttgcc gggccagtca gggcattagc agttatttag cctggtatca gcaaaaacca     120

gggaaagccc ctaagctcct gatctatgct gcatccactt tgcaaagtgg ggtcccatca     180

aggttcagcg gcagtggatc tgggacagaa ttcactctca caatcagcag cctgcagcct     240

gaagattttg caacttatta ctgtcaacag cttaatagtt acccgttcac ttttggccag     300

gggaccaagc tggagatcaa a                                               321


<210>  57
<211>  136
<212>  PRT
<213>  Artificial sequence

<220>
<223>  Human GITR extracellular domain


<400>  57

Gln Arg Pro Thr Gly Gly Pro Gly Cys Gly Pro Gly Arg Leu Leu Leu
1               5                   10                  15


Gly Thr Gly Ala Asp Ala Arg Cys Cys Arg Val His Thr Thr Arg Cys
            20                  25                  30


Cys Arg Asp Tyr Pro Gly Glu Glu Cys Cys Ser Glu Trp Asp Cys Met
        35                  40                  45


Cys Val Gln Pro Glu Phe His Cys Gly Asp Pro Cys Cys Thr Thr Cys
    50                  55                  60


Arg His His Pro Cys Pro Pro Gly Gln Gly Val Gln Ser Gln Gly Lys
65                  70                  75                  80


Phe Ser Phe Gly Phe Gln Cys Ile Asp Cys Ala Ser Gly Thr Phe Ser
                85                  90                  95


Gly Gly His Glu Gly His Cys Lys Pro Trp Thr Asp Cys Thr Gln Phe
            100                 105                 110


Gly Phe Leu Thr Val Phe Pro Gly Asn Lys Thr His Asn Ala Val Cys
        115                 120                 125


Val Pro Gly Ser Pro Pro Ala Glu
    130                 135


<210>  58

<211>  726
<212>  DNA
<213>  Artificial sequence

<220>
<223>  GITR full length


<400>  58
atggcacagc acggggcgat gggcgcgttt cgggccctgt gcggcctggc gctgctgtgc      60

gcgctcagcc tgggtcagcg ccccaccggg ggtcccgggt gcggccctgg gcgcctcctg     120

cttgggacgg gaacggacgc gcgctgctgc cgggttcaca cgacgcgctg ctgccgcgat     180

tacccgggcg aggagtgctg ttccgagtgg gactgcatgt gtgtccagcc tgaattccac     240

tgcggagacc cttgctgcac gacctgccgg caccaccctt gtcccccagg ccagggggta     300

cagtcccagg ggaaattcag ttttggcttc cagtgtatcg actgtgcctc ggggaccttc     360

tccgggggcc acgaaggcca ctgcaaacct tggacagact gcacccagtt cgggtttctc     420

actgtgttcc ctgggaacaa gacccacaac gctgtgtgcg tcccagggtc cccgccggca     480

gagccgcttg ggtggctgac cgtcgtcctc ctggccgtgg ccgcctgcgt cctcctcctg     540

acctcggccc agcttggact gcacatctgg cagctgagga gtcagtgcat gtggccccga     600

gagacccagc tgctgctgga ggtgccgccg tcgaccgaag acgccagaag ctgccagttc     660

cccgaggaag agcggggcga gcgatcggca gaggagaagg ggcggctggg agacctgtgg     720

gtgtga                                                                726